(19) Europäisches Patentamt / European Patent Office / Office européen des brevets

(11) EP 1 481 730 B1

(12) **FASCICULE DE BREVET EUROPEEN**

(45) Date de publication et mention de la délivrance du brevet:
**23.01.2008 Bulletin 2008/04**

(51) Int Cl.:
***B01J 29/70*** *(2006.01)* ***B01J 37/06*** *(2006.01)*
***B01J 38/60*** *(2006.01)* ***C07C 37/60*** *(2006.01)*

(21) Numéro de dépôt: **04291358.2**

(22) Date de dépôt: **28.05.2004**

(54) **Procédé de préparation de composés phénoliques en mettant en oeuvre Zéolite Beta de type protonique**

Verfahren zur Herstellung von Phenolverbindungen mit Zeolith Beta vom Proton-Typ

Process for the production of phenolic compounds using zeolite beta of the proton-type,

(84) Etats contractants désignés:
**FR IT**

(30) Priorité: **30.05.2003 JP 2003153842**
**30.07.2003 JP 2003282286**
**01.09.2003 JP 2003308366**

(43) Date de publication de la demande:
**01.12.2004 Bulletin 2004/49**

(73) Titulaire: **Ube Industries, Ltd.**
**Ube-Shi,**
**Yamaguchi 755-8633 (JP)**

(72) Inventeurs:
- **Kanougi, Tomonori, c/o Ube Industries, Ltd. Ichihara-shi, Chiba 290-0045 (JP)**
- **Atoguchi, Takashi, c/o Ube Industries, Ltd. Ichihara-shi, Chiba 290-0045 (JP)**
- **Yao, Shigeru, c/o Ube Industries, Ltd. Ichihara-shi, Chiba 290-0045 (JP)**

(74) Mandataire: **Goulard, Sophie et al**
**Cabinet ORES**
**36, rue de St Pétersbourg**
**75008 Paris Cedex (FR)**

(56) Documents cités:
**FR-A- 2 693 457** **US-A- 6 025 293**
**US-B2- 6 441 250**

- **ROBERGE D M ET AL: "DEALUMINATION OF ZEOLITE BETA BY ACID LEACHING: A NEW INSIGHT WITH TWO-DIMENSIONAL MULTI-QUANTUM AND CROSS POLARIZATION 27AL MAS NMR" PHYSICAL CHEMISTRY CHEMICAL PHYSICS, ROYAL SOCIETY OF CHEMISTRY, CAMBRIDGE, GB, vol. 4, no. 13, 1 juillet 2002 (2002-07-01), pages 3128-3135, XP001184429 ISSN: 1463-9076**
- **CAMILOTI A M ET AL: "Acidity of Beta zeolite determined by TPD of ammonia and ethylbenzene disproportionation" APPLIED CATALYSIS A: GENERAL, ELSEVIER SCIENCE, AMSTERDAM, NL, vol. 182, no. 1, 7 juin 1999 (1999-06-07), pages 107-113, XP004272027 ISSN: 0926-860X**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 481 730 B1

## Description

La présente invention est relative à un procédé de préparation d'un composé phénolique par oxydation d'un composé benzénique tel qu'un composé phénolique ou un phényléther, etc..., par un peroxyde en présence d'une zéolite β de type protonique et d'une cétone ou d'un acide carboxylique.

Parmi les hydroxyphényléthers représentés par la formule (a) ci-dessous, obtenus par un procédé de préparation d'un composé phénolique de la présente invention, l'o-méthoxyphénol par exemple (guaiacol), dans lequel $R_1$ est un radical méthyle, a été utilisé comme matériau de départ pour des médicaments ou des parfums (13901 Chemicals, publié par "The Chemical Daily Co., Ltd", Japon, 2001, page 653), et le p-méthoxyphénol est un composé important utilisé comme antioxydant ou comme matériau de départ pour un médicament, etc... (Publication de la demande de brevet japonais N° Hei 9-151151).

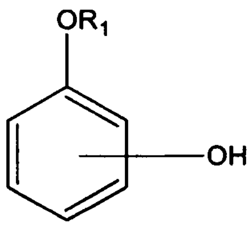 (a)

dans laquelle $R_1$ représente un radical alkyle ayant de 1 à 5 atomes de carbone.

Parmi les techniques connues pour produire un composé phénolique dihydroxylé par oxydation d'un composé phénolique en utilisant une zéolite comme catalyseur, le brevet US 3,580,956 décrit par exemple un procédé dans lequel on utilise de la faujasite ou de la mordénite contenant un métal de terres rares, et le brevet US 4,578,521 décrit par exemple un procédé utilisant une zéolite protonique de type ZSM-5. De même, dans la demande de brevet français FR°2 693 457, on décrit une zéolite naturelle telle que la chabazite, etc..., ou une zéolithe synthétique telle que US-Y, ZSM-5, etc... Cependant, dans ces techniques, le rendement en produit visé n'est pas suffisant. De même, il n'existe aucune description concernant une relation entre la puissance d'un site acide existant sur ces zéolites et une activité catalytique dans la production d'un phénol dihydroxylé.

Dans "Advances in Catalysis", volume 41 (1996), pages 253-334, il est décrit un exemple utilisant la TS-1 (du titane est présent dans le réseau de la ZSM-5). Cependant, comme indiqué dans Accounts of Chemical Research, 31 (8) 1998, pages 485-493, la TS-1 présente fréquemment des problèmes de reproductibilité de préparation, et également, le rendement en produit visé n'est pas suffisant.

De même, dans "Journal of Physical Chemistry", volume 203 (2001), pages 201-212, on décrit une comparaison de la zéolite β contenant du titane dans le réseau avec la TS-1, mais pour la TS-1, son rendement basé sur une quantité de peroxyde d'hydrogène comme standard est de 71,5 % et pour la zéolite β contenant du titane, il est de 62,8 % de sorte que leurs rendements ne sont pas suffisants.

Dans le brevet U.S. 6,441,250 et dans la demande de brevet japonais n° 2003-26623, on décrit un exemple qui utilise de la zéolite β et on décrit en outre que l'on utilise une zéolite β dans laquelle un ion de métal alcalin a été introduit ; le rendement en composé phénolique dihydroxylé est alors amélioré. Cependant, dans la zéolite β décrite dans ces références, la dépendance à la température par rapport à un rendement de réaction est élevée et le rendement obtenu à une température inférieure à 90°C ou moins est insuffisant. Ainsi, l'intervalle de température est limité lorsque la réaction est réalisée dans la pratique, de sorte qu'une amélioration supplémentaire est souhaitée.

D'autre part, au cours d'une réaction de production d'un hydroxyphényléther en une seule étape par oxydation d'un phényléther, l'o-hydroxyphényléther, dans lequel la position ortho a été oxydée, et le p-hydroxyphényléther, dans lequel la position para a été oxydée, sont, en général, produits simultanément (voir par exemple, Journal of American Chemical Society, 1981, 103, pages 3045-3049 ; Journal of Chemical Society Perkin Transition, 1990, 1(6), pages 863-867 ; Journal of Chemical Society, Chemical Communications, 1995, 3, pages 349-350). La capacité d'utilisation en industrie de l'hydroxyphényléther peut varier en fonction de la façon dont il est substitué, en particulier les composés substitués en position ortho sont nettement différents des composés substitués en position para. Par exemple, l'o-méthoxyphénol est une matière première importante pour des médicaments ou des parfums, et le p-méthoxyphénol est une matière première importante comme antioxydant (voir la demande de brevet japonais n° Hei 9-151151 et "13901 Chemicals", publié par The Chemical Daily Co., Ltd, Japon, 2001, page 653).

Ainsi, lorsque ces composés sont produits en combinaison, il existe des problèmes non seulement au niveau de leur rendement, mais également du rapport formé du composé substitué en position ortho au composé substitué en

position para. Par conséquent, les techniques respectives pour produire de manière prédominante le composé substitué en position ortho ou le composé substitué en position para sont chacune importantes.

A titre de technique permettant de produire de manière prédominante le composé substitué en position ortho ou le matériau substitué en position para par oxydation d'un phényléther pour conduire à un hydroxyphényléther en une seule étape, on peut mentionner ce qui suit.

Parmi celles-ci, et comme technique utilisant un catalyseur homogène, "13901 Chemicals", publié par The Chemical Daily Co., Ltd, Japon, 2001, page 653, décrit un procédé de préparation de méthoxyphénol selon lequel on oxyde l'anisole par du peroxyde d'hydrogène en présence de polynitroporphyrine de manganèse à titre de catalyseur. Selon cette méthode, le rendement en méthoxyphénol, sur la base de la quantité de peroxyde d'hydrogène utilisée, est de 98 %. Cependant, alors que cette méthode présente une sélectivité élevée pour la position para (un rapport de formation du composé substitué en position ortho/composé substitué en position para (ci-après indiqué rapport o/p) de 0,11), elle nécessite l'utilisation de grandes quantités de catalyseur et pose des problèmes de récupération et de réutilisation du catalyseur, de sorte qu'elle est difficile à mettre en oeuvre à l'échelle industrielle.

Dans Organic Preparations and Procedures Int., 2000, 32, pages 373-405, il est décrit un procédé de préparation du méthoxyphénol à partir de l'anisole en utilisant le peroxyde d'hydrogène comme agent oxydant et le nitrate de cuivre comme catalyseur. Dans ce procédé, la réaction est réalisée par contrôle du pH du système réactionnel avec un tampon phosphate, ce par quoi on atteint un rendement élevé de 94 % en méthoxyphénol par rapport à la quantité d'anisole utilisée ainsi qu'une sélectivité élevée pour la position para avec un rapport o/p de 0,12. Cependant, c'est une réaction en système homogène, de sorte que la récupération du catalyseur est difficile et une quantité extrêmement élevée de solvant est nécessaire, de sorte que ce procédé, notamment en raison de l'étape de récupération, est compliqué et pénible à mettre en oeuvre à l'échelle industrielle.

Comme technique utilisant un catalyseur hétérogène, le Journal of Chemical Society, Chemical Communications, 1995, 3, pages 349-350, décrit un procédé de préparation de méthoxyphénol qui comprend l'oxydation de l'anisole avec du peroxyde d'hydrogène en utilisant la TS-1 contenant du titane dans le réseau. Selon ce procédé, le rendement en méthoxyphénol sur la base de l'anisole est de 67 %, et le rapport o/p est égal à 0,35. Comme indiqué dans la littérature mentionnée ci-dessus, la préparation de la TS-1 présente souvent un problème de reproductibilité et le rendement de réaction n'est pas suffisant.

La demande de brevet japonais n° Hei 3-128336 décrit un procédé de préparation du méthoxyphénol par oxydation de l'anisole en utilisant un système catalytique comprenant un sel de métal alcalin d'un acide protonique comme l'acide sulfurique, etc... et un oxyacide de phosphore comme l'acide orthophosphorique, etc..., mais il n'y a aucune description d'une zéolite.

La demande de brevet français n° 2 693 457 décrit un procédé de préparation du méthoxyphénol par oxydation de l'anisole en utilisant de la zéolite US-Y à titre de zéolite de type protonique en présence d'une cétone ; mais il n'y a aucune description d'une zéolite β de type protonique, et ses propriétés catalytiques ne sont pas divulguées. De même, un test effectué par les inventeurs montre que le rendement obtenu en méthoxyphénol est de 47,1 % (rapport o/p = 2,86) de sorte qu'il est insuffisant (voir exemple comparatif 7 ci-après).

D'autre part, par exemple dans Journal of American Chemical Society, 1981, 103, pages 3045-3049, il est décrit un procédé de préparation du méthoxyphénol à partir d'anisole par photooxydation en utilisant un peroxyde d'un composé de type azo comme agent oxydant. Dans ce procédé, on forme de manière prédominante, un composé substitué en position ortho (avec un rapport o/p = 1,78), mais le rendement en composé attendu sur la base de la quantité d'agent oxydant utilisée est d'environ 30 %. De même, dans Journal of Chemical Society Perkin Transition, 1990, 1(6), pages 863-867 ; on décrit un procédé de préparation du méthoxyphénol à partir de l'anisole par photooxydation en utilisant un composé N-oxyde ayant une structure hétérocyclique comme agent oxydant. Selon ce procédé, on forme de manière prédominante un composé substitué en position ortho (rapport o/p = 1,47), mais le rendement en méthoxyphénol sur la base de la quantité d'agent oxydant utilisée est d'environ 42 % comme rendement global. Ces techniques présentent l'avantage de n'utiliser aucun catalyseur, mais conduisent au produit attendu avec un rendement insuffisant tout en mettant en oeuvre un agent oxydant onéreux.

Comme décrit ci-dessus, et en ce qui concerne la technique permettant d'oxyder, de façon prédominante, un composé substitué en position ortho, il existe des rapports selon lesquels le rendement en hydroxyphényléther sur base de la quantité d'agent oxydant utilisée est d'environ 40 %. Aucune technique permettant d'obtenir de l'hydroxyphényléther avec un rendement plus élevé n'a encore été trouvée.

Enfin, l'article de Roberge et al., Physical Chemistry Chemical Physics, 2002, 13, pages 3128-3135 décrit un procédé de désalumination d'une zéolite P par lixiviation acide, et un procédé de préparation d'une composition catalytique de zéolite supportée comprenant notamment une étape de traitement par de l'acide oxalique est décrit dans le Brevet U.S. 6,025,293.

Un objet de la présente invention consiste à proposer un procédé de préparation industriel d'un composé phénolique substitué en position ortho et/ou substitué en position para, par oxydation d'un composé benzénique tel qu'un composé phénolique ou un composé phényléther, etc..., avec un peroxyde en utilisant une zéolite β de type

protonique, qui peut être facilement et simplement préparée et qui présente une réactivité élevée à plus faible température, pour conduire à un composé phénolique avec un rendement élevé.

Les inventeurs ont cherché à résoudre les problèmes indiqués ci-dessus et ont découvert, comme résultat, un procédé industriel de préparation d'un composé phénolique substitué en position ortho et/ou substitué en position para par oxydation d'un composé benzénique tel qu'un composé phénolique ou un composé phényléther, etc..., avec un peroxyde en utilisant une zéolite β de type protonique, pour conduire à un composé phénolique substitué en position ortho et/ou substitué en position para avec un rendement élevé.

Ainsi, la présente invention est la suivante.

La présente invention a pour objet un procédé de préparation d'au moins un des composés phénoliques choisis dans le groupe consistant en un composé représenté par les formules (3) et (4) suivantes :

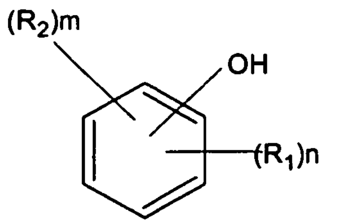

(3)

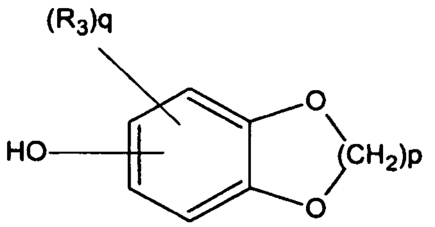

(4)

dans lesquelles :

- $R_1$ représente un radical hydroxyle, un radical alkyle linéaire ou ramifié ayant de 1 à 6 atomes de carbone ou un radical alcoxy linéaire ou ramifié ayant de 1 à 6 atomes de carbone ;
- $R_2$ représente un atome d'hydrogène, un radical alkyle linéaire ou ramifié ayant de 1 à 6 atomes de carbone, un radical alcoxy linéaire ou ramifié ayant de 1 à 6 atomes de carbone ou un atome d'halogène ;
- n est un nombre entier allant de 0 à 5 ;
- m est un nombre entier allant de 0 à 5 ;
- la somme n + m va de 0 à 5 ;
- en cas de pluralité de radicaux $R_1$ et/ou $R_2$, ceux-ci peuvent être identiques ou différents ;
- $R_3$ représente un atome d'hydrogène, un radical alkyle linéaire ou ramifié ayant de 1 à 6 atomes de carbone, un radical alcoxy linéaire ou ramifié ayant de 1 à 6 atomes de carbone ou un atome d'halogène,
- p est un nombre entier allant de 1 à 2 et
- q est un nombre entier allant de 0 à 3 ;

qui comprend l'oxydation d'au moins un des composés benzéniques représentés par les formules (1) et (2) suivantes :

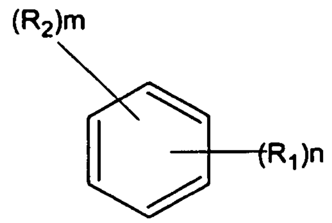

(1)

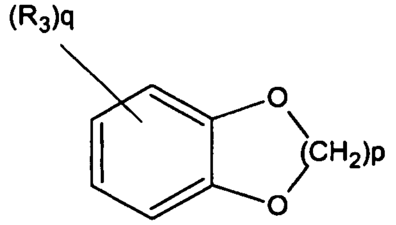

(2)

dans lesquelles $R_1$, $R_2$, $R_3$, n, m, p et q ont les mêmes significations que celles définies ci-dessus pour les composés de formules (3) et (4),
avec un peroxyde en présence d'une zéolite β de type protonique comprenant un site acide présentant un pic de désorption dans une gamme de ± 100°C avec un centre à 330°C, selon un spectre mesuré par la méthode de désorption à l'ammoniaque à température programmée (TPD-$NH_3$), et une quantité de site acide fort présentant un pic de désorption à une température d'au moins 500°C inférieure ou égale à 2,5 μmole/g, et en présence d'une cétone ou d'un acide carboxylique.

Brève description des dessins :

- La Figure 1 est un spectre (dessin schématique) de la zéolite β de type protonique obtenu par la méthode de désorption à l'ammoniaque à température programmée (TPD-$NH_3$).
- La Figure 2 est un spectre de H/β (38) (courbe haute) et de H/β $HNO_3$ (38) (courbe basse) obtenu par la méthode de désorption à l'ammoniaque à température programmée (TPD-$NH_3$).
- La Figure 3 est un spectre IR-TF de H/β (38) et de H/β $HNO_3$ (38).

Des modes de réalisation préférés de la présente Invention vont maintenant être décrits.

La zéolite β de type protonique conforme à la présente invention peut être obtenue par immersion de zéolite β dans une solution acide aqueuse, puis après sa récupération de la zéolite β ainsi traitée de la solution acide aqueuse, on procède, dans cet ordre, à son lavage, son séchage et à sa calcination.

A titre de zéolite β, on utilise de préférence une zéolite β ayant un rapport atomique Al:Si compris dans l'intervalle allant de 1:25 à 1:10 000. Incidemment, ce rapport peut être contrôlé par la composition en Al et Si de composés utilisés à titre de matière première pour la zéolite β, comme par exemple par la composition en silicate de sodium et en aluminate de sodium.

A titre de zéolite β, on peut utiliser une zéolite β de type protonique, une zéolite β de type ammonium obtenue par échange d'ions, une zéolite β de type métal alcalin obtenue par échange d'ions ou des zéolites β similaires qui n'ont pas été soumises à un traitement avec une solution acide aqueuse selon la présente invention.

La zéolite β de type protonique peut être préparée par la méthode décrite dans Journal of Physical Chemistry, 2000, 104, pages 2853 à 2859. Par exemple, selon cette méthode, une zéolite β (ci-après, indiquée zéolite β TS (telle que synthétisée) obtenue en soumettant une source de silicium (gel de silice, etc...), une source d'aluminium (aluminate de sodium, pseudo-boehmite, etc.) et un alkylammonium (hydroxyde de tétraalkylammonium, etc...), comme matériaux de départ à une synthèse hydrothermique et à une calcination, est soumise à un traitement thermique dans une solution aqueuse contenant des ions ammonium (concentration en sel d'ammonium : 0,1 à 40 % en poids) comme le nitrate d'ammonium, le chlorure d'ammonium, etc..., à une température de 20 à 120°C, pendant 1 à 20 heures, puis lavée avec de l'eau déminéralisée, etc..., séchée à une température de 20 à 150°C, puis calcinée à une température de 300 à

650°C pendant 1 à 10 heures. Selon une autre méthode préférée, la zéolite β de type protonique peut être obtenue par immersion de zéolite β TS telle que définie ci-dessus dans une solution aqueuse d'acide chlorhydrique 0,01 N à 1 N, traitement à une température allant de la température ambiante à 100°C pendant plusieurs minutes à plusieurs jours, puis lavage avec de l'eau déminéralisée, etc..., séchage à une température allant de la température ambiante à 150°C et calcination à une température comprise entre 150°C et 650°C.

La zéolite β TS peut être préparée par les méthodes décrites dans les documents de l'art antérieur mentionnés ci-dessus ou bien être directement achetée dans le commerce.

La teneur en aluminium (aluminium dans le squelette) dans la zéolite β TS se situe de préférence, à un rapport dans lequel Al:Si (rapport atomique) est dans l'intervalle allant de 1:25 à 1:10 000. En outre, ce rapport peut être contrôlé en modifiant la composition d'un composé Si et d'un composé Al, qui sont les matériaux de départ de la zéolite β TS, par exemple le silicate de sodium et l'aluminate de sodium.

A titre de zéolite β de type ammonium obtenue par échange d'ions, on peut utiliser un composé disponible dans le commerce. La zéolite β de type métal alcalin obtenue par échange d'ions peut être préparée selon le procédé décrit dans « Catalyst Préparation Chemistry » publié par Kodansha, Japon (1980), pages 61-73, dans lequel on immerge une zéolite β de type protonique dans la solution aqueuse contenant un ion métallique et telle que décrite ci-dessus, comme par exemple un nitrate, un chlorhydrate ou un sulfate du métal alcalin.

Parmi ces zéolites β, on préfère utiliser une zéolite β protonique, n'ayant pas été soumise à un traitement avec une solution acide aqueuse selon la présente invention.

La nature de l'acide à utiliser dans la solution acide aqueuse conformément à la présente invention n'est pas spécifiquement limitée, et on peut mentionner un acide organique tel qu'un acide carboxylique aliphatique (acide formique, acide acétique, acide propionique, acide citrique, etc...), un acide carboxylique aromatique (acide benzoïque, etc...), un acide aminé (acide aspartique, acide glutamique, etc...) ou un acide sulfonique (acide trifluorométhylsulfonique, etc...), etc., ou bien un acide inorganique (acide nitrique, acide phosphorique, acide fluorhydrique, acide chlorhydrique, acide sulfurique, etc...). On préfère utiliser un acide inorganique, et tout particulièrement l'acide nitrique.

Parmi les solutions acides aqueuses mentionnées ci-dessus, on préfère celles dont le pH est ajusté à une valeur de 0,01 à 6,0, de préférence de 0,5 à 4,0. De même, la quantité de solution aqueuse acide à utiliser n'est pas spécifiquement critique à partir du moment où ladite zéolite β est suffisamment immergée.

La température à laquelle la zéolite β est immergée invention dans la solution acide aqueuse conformément à la présente invention se situe, de préférence, dans l'intervalle allant de la température ambiante à 120°C, de manière plus préférée de 40 à 90°C.

Egalement, on préfère une durée d'immersion allant de 1 à 12 heures.

L'opération d'immersion est de préférence réalisée, sous agitation, mais même lorsqu'on laisse au repos, un effet suffisant peut être obtenu.

Ensuite, la zéolite β immergée dans la solution acide aqueuse est récupérée et lavée avec de l'eau. Ici, une méthode de récupération particulière n'est pas obligatoire, et par exemple, on la récupération est réalisée par un procédé tel que la filtration, etc... L'eau à utiliser pour le lavage est de préférence de l'eau déminéralisée.

La zéolite β lavée telle que décrite ci-dessus est alors séchée.

L'intervalle de température pour le séchage est de préférence de 90 à 150°C. La duré du séchage peut varier en fonction de la quantité de zéolite β telle que décrite ci-dessus ; elle est de préférence de 1 heure à 2 jours.

Après le séchage, la zéolite β telle que décrite ci-dessus est soumise à un traitement de calcination. La température de calcination se situe, de préférence, de 350 à 950°C, de manière plus préférée, de 450 à 850°C.

La durée de la calcination est, de préférence, de 1 à 24 heures.

La zéolite β de type protonique conforme à la présente invention, obtenue en procédant au traitement par la solution acide aqueuse indiqué ci-dessus, a des caractéristiques telles que lorsque l'on compare son spectre mesuré par la méthode de désorption à l'ammoniaque à température programmée (TPD-$NH_3$, vitesse d'augmentation de la température : 20°C/minute, intervalle mesuré : 100 à 700°C) à celui du matériau non traité, on observe un site acide montrant un pic de désorption avec un intervalle de ± 100°C avec un centre à 330°C, et une quantité de site acide fort présentant un pic de désorption à une température supérieure ou égale à 500°C inférieure ou égale à 2,5 μmole/g, de manière plus préférée compris entre 0,0005 μmole/g (calculée à partir de la limite de détection du pic de désorption), et 2,5 μmoles/g inclusivement. En outre, le pic de désorption à une température proche de 200°C d'un matériau non traité dû à de l'ammoniac physiquement adsorbé sur la structure de la zéolite β et basé sur une force de désorption, etc., est nettement diminué par la solution acide aqueuse (voir Fig. 2).

Selon la présente invention, la quantité du site acide fort est calculée à partir de la quantité d'ammoniac désorbée au cours du procédé tel que décrit ci-dessus de désorption à l'ammoniaque à température programmée (TPD-$NH_3$) (voir Microporous and Mesoporous Materials, 2000, 40, pages 271-281).

De même, le calcul de la quantité d'ammoniac désorbé est réalisé selon la méthide de détermination décrite par Keulemans "Gas Chromatography", Reinhold, New York, 1957, page 39 ; un pic de désorption à une température d'au moins 500°C, se situant sur la partie inférieure (basale) d'un pic principal avec un centre à 330°C, est délimité par

une ligne d'extension du pic principal tel qu'indiqué ci-dessus, et quantifié par la surface (voir Fig. 1 et l'équation suivante) :

Quantité de site acide fort = ($NH_3$ – quantité d'ammoniac total désorbé à une température allant de 100°C à 700°C mesurée par TPD) x {(surface de B) / (surface de A + surface de B)},

équation dans laquelle A représente un pic principal avec un centre à 330°C, et B représente un pic de désorption à une température d'au moins 500°C, (voir Fig. 1).

De même, la zéolite β de type protonique conforme à la présente invention est caractérisée par une absorption à 3782 $cm^{-1}$, comme limite de détection ou moins lorsque son spectre IR-TF est comparé à celui de la zéolite non traitée (voir Fig. 3).

Le procédé de préparation d'un composé phénolique substitué en position para et/ou substitué en position ortho à l'aide de la zéolite β de type protonique de la présente invention va maintenant être expliqué.

Dans la formule (1) ci-dessus, $R_1$ représente un radical hydroxyle, un radical alkyle linéaire ou ramifié ayant de 1 à 6 atomes de carbone ou un radical alcoxy linéaire ou ramifié ayant de 1 à 6 atomes de carbone.

A titre de radical alkyle linéaire ou ramifié ayant de 1 à 6 atomes de carbone pour $R_1$, on peut mentionner par exemple, le radical méthyle, le radical éthyle, le radical propyle, le radical butyle, le radical pentyle ou le radical hexyle. En outre, ces radicaux peuvent comprendre les isomères. De même, à titre de radical alcoxy linéaire ou ramifié ayant de 1 à 6 atomes de carbone pour $R_1$, on peut mentionner par exemple, le radical méthoxy, le radical éthoxy, le radical n-propoxy, le radical isopropoxy, le radical n-butoxy, le radical t-butoxy, etc..., ainsi que leurs isomères structuraux.. Dans la formule (1), n est de préférence un nombre entier allant de 0 à 3, et de manière encore plus préférée un nombre entier égal à 0 ou 1.

Dans la formule (1) ci-dessus, lorsque $R_1$ est un radical hydroxyle et n = 1, alors le composé de formule (I) est un composé phénolique.

La zéolite β de type protonique conforme à la présente invention est de préférence utilisée dans la préparation d'un composé phénolique dihydroxylé, qui comprend l'oxydation d'un composé phénolique avec un peroxyde en présence d'une cétone.

A titre de phénol pouvant être utilisé conformément à la présente invention, on peut par exemple mentionner le phénol dans lequel les $R_2$ sont tous des atomes d'hydrogène, un monoalkylphénol monovalent, un phénol halogéné monovalent et un polyalkylphénol monovalent, dans lesquels au moins un des $R_2$ est un radical alkyle linéaire ou ramifié ayant de 1 à 6 atomes de carbone ou un atome d'halogène.

Pour $R_2$, à titre de radical alkyle linéaire ou ramifié ayant de 1 à 6 atomes de carbone ou à titre de radical alcoxy linéaire ou ramifié ayant de 1 à 6 atomes de carbone, on peut indiquer ceux mentionnés pour $R_1$. Dans la formule (1), m est de préférence un nombre entier allant de 0 à 3, de manière encore plus préférée m est un nombre entier égal à 0 ou 1.

Parmi les atomes d'halogène mentionnés pour $R_2$, on peut citer l'atome de fluor, l'atome de chlore, l'atome de brome et l'atome d'iode.

La position du radical alkyle du monoalkylphénol monovalent n'est pas critique tant que ce radical ne participe pas à la réaction. Parmi de tels composés, on peut mentionner par exemple le o-, m- ou p-crésol, le o-, m- ou p-éthylphénol, l'o-propylphénol, le p-*iso*propylphénol, le m-butylphénol, le p-*iso*butylphénol, le p-*t*-butylphénol, le m-*iso*-butylphénol, le p-pentylphénol et le p-hexylphénol.

Comme atome d'halogène du phénol halogéné monovalent, on peut mentionner le fluor, le chlore, le brome et l'iode. Le nombre et la position des atomes d'halogène ne sont pas critiques tant que ces atomes ne participent pas à la réaction. Parmi ces composés, on peut mentionner par exemple, le o-, m- ou p-fluorophénol, le o-, m- ou p-chlorophénol, le o-, m- ou p-bromophénol, le o-, m- ou p-iodophénol, le 2,3-, 2,4-, 2,5-, 2,6-, 3,4- ou 3,5-dichlorophénol, le 2,3-, 2,4-, 2,5-, 2,6-, 3,4- ou 3,5-dibromophénol, le 2,3,4-, 2,3,5-, 2,3,6-, 2,4,5-, 2,4,6- ou 3,4,5-trichlorophénol.

En ce qui concerne le polyalkylphénol monovalent, dans lequel plusieurs radicaux alkyle comme $R_2$ sont présents, le nombre et la position des radicaux alkyle ne sont pas critique tant que ces radicaux ne participent pas à la réaction. De tels composés peuvent comprendre par exemple, le 2,3-, 2,4-, 2,5-, 2,6-, 3,4- ou 3,5-diméthylphénol, le 2,3,4-, 2,3,5-, 2,3,6-, ou 3,4,5-triméthylphénol, le 2-éthyl-3-méthylphénol, le 3-t-butyl-4-méthylphénol, le 2-isopropyl-5-méthylphénol, le 2-pentyl-6-méthylphénol et le 3-hexyl-5-méthylphénol.

En ce qui concerne le polyalkylphénol monovalent, dans lequel plusieurs dont au moins 2 radicaux alkyle, radicaux alcoxy et atomes d'halogène comme $R_2$ sont présents, le nombre et la position des radicaux alkyle, le nombre et la position des radicaux alcoxy et le nombre et la position des atomes d'halogène ne sont pas critiques tant qu'ils ne participent pas à la réaction. De tels composés peuvent comprendre par exemple, le 2-méthyl-3-fluorophénol, le 2-méthyl-3-chlorophénol, le 2-méthyl-3-bromophénol, le 2-méthyl-3-iodophénol, le 2-méthyl-4-fluorophénol, le 2-méthyl-4-chlorophénol, le 2-méthyl-4-bromophénol, le 2-méthyl-4-iodophénol, le 4-chloro-2-isopropyl-5-méthylphénol, le 4-chloro-3,5-diméthylphénol, le 4-bromo-2,6-diméthylphénol, le 4-bromo-3,5-diméthylphénol, le 2-méthoxy-3-fluorophénol, le 2-méthoxy-3-chlorophénol, le 2-méthoxy-3-bromophénol, le 2-méthoxy-3-iodophénol, le 2-méthoxy-4-fluorophénol, le 2-méthoxy-4-chlorophénol, le 2-méthoxy-4-bromophénol, le 2-méthoxy-4-iodophénol, le 4-chloro-2-isopropyl-5-méthoxyphénol, le 4-chloro-3,5-diméthoxyphénol, le 4-bromo-2,6-diméthoxyphénol et le 4-bromo-3,5-diméthoxyphénol.

A titre de cétone pouvant être utilisée selon le procédé de préparation d'un phénol dihydroxylé à l'aide de la zéolite β de type protonique conformément la présente invention, on peut mentionner par exemple, une monocétone ou une dicétone. Comme monocétone, on peut mentionner une monocétone non cyclique ou cyclique. Comme monocétone non cyclique, on peut par exemple citer une monocétone aliphatique, linéaire ou ramifiée, ayant de 3 à 20 atomes de carbone, de préférence de 3 à 10 atomes de carbone ou une monocétone aromatique. Le ou les atomes d'hydrogène de ces composés peuvent être substitués par des atomes d'halogène (atome de fluor, atome de chlore, atome de brome ou atome d'iode). Le nombre et la position des atomes d'halogène ne sont pas critiques, tant qu'ils ne participent pas à la réaction.

Comme monocétone aliphatique linéaire, on peut mentionner par exemple, l'acétone, la méthyléthylcétone, la 2-pentanone, la 3-pentanone, la 2-hexanone, la 2-heptanone, la 3-heptanone, la 4-heptanone, la 2-octanone, la 2-nonanone, la 3-décanone, la 6-undécanone, la 3-tridécanone, la 7-tridécanone, la 2-tétradécanone, la 2-pentadécanone, la 2-hexadécanone, la 2-heptadécanone, la 3-octadécanone, la 4-nonadécanone, la 1-chloro-2-propanone, la 1-chloro-3-heptanone et la 1-bromo-3-heptanone.

Comme monocétone aliphatique ramifiée, on peut mentionner par exemple, la 3-méthyl-2-butanone, la 3-méthyl-2-pentanone, la 4-méthyl-2-pentanone, la 3,3-diméthyl-2-butanone, la 2,4-méthyl-3-pentanone, la 6-méthyl-2-heptanone, la 2,6-méthyl-4-heptanone et la 2,2,4,4-tétraméthyl-3-heptanone. A titre de monocétone aromatique, on peut mentionner par exemple, l'acétophénone, la benzophénone, la 1-phényl-3-propanone, la 1-phényl-2-butanone, la 1-phényl-3-butanone, la 1-phényl-3-pentanone et la 1,3-diphényl-2-propanone.

A titre de monocétone cyclique, on peut par exemple mentionner une monocétone cycloalkylique ayant de 5 à 12 atomes de carbone. Le ou les atomes d'hydrogène de ces composés peuvent être remplacés par des substituants tels que par un atome d'halogène (atome de fluor, atome de chlore, atome de brome ou atome d'iode), ou par un radical alkyle linaire ou ramifié ayant de 1 à 6 atomes de carbone. Le nombre et la position des substituants ne sont pas critiques, tant qu'ils ne participent pas à la réaction. De tels composés peuvent comprendre par exemple la cyclopentanone, la cyclohexanone, la cyclododécanone, le 2-chlorocyclohexanone, la 2-éthyl-1-cyclopentanone et la 2-méthyl-1-cyclohexanone.

A titre de dicétone, on peut mentionner une dicétone non cyclique ou cyclique. A titre de dicétone non cyclique, on peut par exemple mentionner une dicétone aliphatique linéaire ou ramifiée ayant de 5 à 21 atomes de carbone, de préférence de 5 à 12 atomes de carbone, ou une dicétone aromatique. Un ou des atomes d'hydrogène de ces composés peuvent être substitués par des atomes d'halogène (atome de fluor, atome de chlore, atome de brome ou atome d'iode). Le nombre et la position des atomes d'halogène ne sont pas critiques, tant qu'ils ne participent pas à la réaction. A titre de dicétone aliphatique linéaire, on peut par exemple mentionner la 2,3-butanedione, la 2,4-pentanedione et la 2,5-hexanedione peuvent être mentionnées. A titre de dicétone aliphatique ramifiée, on peut par exemple mentionner la 2,5-diméthyl-3,4-hexanedione. A titre de dicétone aromatique, on peut par exemple mentionner la 1,2-diphényléthane-1,2-dione.

A titre de dicétone cyclique, on peut par exemple mentionner une dicétone cyclique ayant de 5 à 12 atomes de carbone. Le ou les atomes d'hydrogène de ces composés peuvent être remplacés par des substituants tels qu'un atome d'halogène (atome de fluor, atome de chlore, atome de brome ou atome d'iode), un radical alkyle linaire ou ramifié ayant de 1 à 6 atomes de carbone ou similaire. Le nombre et la position des substituants ne sont pas critiques, tant qu'ils ne participent pas à la réaction. Comme dicétone cyclique, on peut par exemple mentionner la 1,4-cyclohexanedione.

Les cétones utilisées dans le procédé de préparation d'un phénol dihydroxylé en utilisant la zéolite β de type protonique conformément à la présente invention, sont de préférence une monocétone aliphatique linéaire ou ramifiée ou une monocétone cyclique, de manière encore plus préférée, une monocétone aliphatique linéaire ou ramifiée et de manière encore plus particulièrement préférée, la 4-méthyl-2-pentanone ou la 3-pentanone.

La quantité de cétone à utiliser est, de préférence, un rapport dans lequel le rapport molaire de la cétone sur la quantité de peroxyde (cétone:peroxyde) est de 0,2:1 à 5:1.

Au cours du procédé de préparation d'un phénol dihydroxylé en utilisant la zéolite β de type protonique de la présente invention, on peut à titre optionnel ajouter une faible quantité d'acide phosphorique pour éviter les effets néfastes exercés sur la réaction par les ions métalliques dissous tels qu'une faible quantité d'ions fer, etc..., bien que cela ne soit pas essentiel. A titre d'acide phosphorique, on peut mentionner l'acide orthophosphorique, l'acide pyrrophosphorique, l'acide métaphosphorique, l'acide triphosphorique, l'acide tetraphosphorique, l'acide polyphosphorique, l'anhydride phosphorique et une solution aqueuse d'acide phosphorique ; on utilise de préférence une solution aqueuse

d'acide phosphorique. La concentration de la solution aqueuse d'acide phosphorique est de préférence comprise entre 0,001 et 100 % en poids inclusivement.

La quantité d'acide phosphorique à utiliser se situe de préférence dans le rapport dans lequel le rapport pondéral de l'acide phosphorique à la quantité de phénol (acide phosphorique:phénol) est de 0,0001:1 à 0,05:1.

Le peroxyde à utiliser conformément au procédé de préparation d'un phénol dihydroxylé en utilisant la zéolite β de type protonique selon la présente invention, peut être un peroxyde inorganique comme le peroxyde d'hydrogène, etc., ou un peroxyde organique comme un peroxyde de cétone, un peracide carboxylique aliphatique, etc.

A titre de peroxyde de cétone, on peut par exemple mentionner de préférence, un peroxyde de dialkylcétone ayant de préférence de 3 à 20 atomes de carbone, et de manière plus préférée, de 3 à 10 atomes de carbone. Parmi ces composés, on peut mentionner par exemple, le peroxyde de la diméthylcétone, le peroxyde de la diéthylcétone, le peroxyde de la méthyléthylcétone, le peroxyde de la méthyl-n-propylcétone, le peroxyde de la méthylisopropylcétone, le peroxyde de la méthylisobutylcétone et leurs analogues.

A titre de peracide carboxylique aliphatique, on peut mentionner l'acide peracétique, l'acide perpropionique, etc...

En ce qui concerne peroxyde d'hydrogène, on peut utiliser du peroxyde d'hydrogène aqueux ayant une concentration d'au moins 0,1 % en poids, de préférence de 0,1 à 90 % en poids ; le peroxyde d'hydrogène à une concentration de 30 à 80 % en poids est utilisé de préférence.

A titre de peroxyde à utiliser dans le processus de préparation d'un phénol dihydroxylé en utilisant la zéolite β de type protonique conformément à la présente invention, on préfère utiliser le peroxyde d'hydrogène ou un peroxyde de cétone. Ce peroxyde de cétone peut être synthétisé par mise en contact d'une cétone avec du peroxyde d'hydrogène, et la cétone à utiliser ici est la même que celles mentionnées ci-dessus.

La quantité de peroxyde à utiliser se situe de préférence, dans la gamme dans laquelle le rapport molaire du peroxyde à la quantité de phénol (peroxyde:phénol) est de 1:1 à 1:100, de manière plus préférée dans la gamme allant de 1:5 à 1:20.

Par ailleurs, dans la formule (1), lorsque $R_1$ est un radical alkyle linéaire ou ramifié ayant de 1 à 6 atomes de carbone ou un radical alcoxy linéaire ou ramifié ayant de 1 à 6 atomes, et $R_2$ est un atome d'hydrogène, un radical alkyle linéaire ou ramifié ayant de 1 à 6 atomes de carbone ou un atome d'halogène, le procédé devient une réaction dans laquelle le composé benzénique substitué est oxydé pour conduire à un composé phénolique substitué. Selon la présente invention, pour préparer un composé phénolique substitué en position para, de manière prédominante, le composé benzénique représenté par la formule (1) est oxydé par un peroxyde en présence de la zéolite β de type protonique, de la cétone et de l'acide phosphorique mentionnés ci-dessus. De même, pour préparer un composé phénolique substitué en position ortho, de manière prédominante, le composé benzénique représenté par la formule (1) est oxydé par un peroxyde en présence de la zéolite β de type protonique, de l'acide carboxylique et de l'acide phosphorique, ce par quoi le composé attendu peut être obtenu.

Dans la formule (1) ci-dessus, lorsque $R_1$ est un radical alkyle linéaire ou ramifié ayant de 1 à 6 atomes de carbone ou un radical alcoxy linéaire ou ramifié ayant de 1 à 6 atomes, on préfère que n soit égal à 1 et que les $R_2$ soient tous des atomes d'hydrogène.

A titre de composés benzéniques substitués mentionnés ci-dessus, on peut mentionner un composé benzénique substitué par un radical alkyle, un composé benzénique substitué par un radical alkyle et un atome d'halogène, un composé benzénique substitué par un radical alcoxy, un composé benzénique substitué par un radical alcoxy et un atome d'halogène, et leurs analogues structuraux. Comme composé benzénique substitué par un radical alkyle, on peut mentionner par exemple, le méthylbenzène, l'éthylbenzène, le n-propylbenzène, l'isopropylbenzène, le n-butylbenzène, le t-butylbenzène, le o-, m- ou p-xylène, le 1,2,3-, 1,2,4- ou 1,3,5-triméthylbenzène, et le 1,2,3,4-, 1,2,3,5- ou 1,2,4,5-tétraméthylbenzène, etc... A titre de composé benzénique substitué par un radical alkyle et par un atome d'halogène, on peut mentionner par exemple, le 2-chlorotoluène, le 3-chlorotoluène, le 4-chlorotoluène, le 2-fluorotoluène, le 3-fluorotoluène, le 4-fluoro-toluène, le 2-bromotoluène, le 3-bromotoluène, le 4-bromotoluène, etc...

A titre de composé benzénique substitué par un radical alcoxy, on peut mentionner par exemple, le méthoxybenzène (anisole), l'éthoxybenzène, le *n*-propoxybenzène, l'*iso*propoxybenzène, le *n*-butoxybenzène, le *t*-butoxybenzène, le o-, m- ou p-diméthoxybenzène, le 1,2,3-, 1,2,4- ou 1,3,5-triméthoxybenzène, le 1,2,3,4-, 1,2,3,5- ou 1,2,4,5-tétraméthoxybenzène, etc... A titre de composé benzénique substitué par un radical alcoxy et par un atome d'halogène, on peut mentionner par exemple, le 2-chloroanisole, le 3-chloroanisole, le 4-chloroanisole, le 2-fluoroanisole, le 3-fluoroanisole, le 4-fluoroanisole, le 2-bromoanisole, le 3-bromoanisole, le 4-bromoanisole, etc...

En ce qui concerne le composé représenté par la formule (2) indiquée ci-dessus, $R_3$ est identique à $R_2$ tel que mentionné ci-dessus. q est de préférence un nombre entier allant de 0 à 2, de manière plus préférée, un nombre entier égal à 0 ou 1.

De même, à titre de composé représenté par la formule (2) indiquée ci-dessus, on peut en particulier mentionner le 1,2-méthylènedioxybenzène (1,3-benzodioxole), le 3-méthyl-1,2-méthylènedioxybenzène, le 3-chloro-1,2-méthylènedioxybenzène, etc... lorsque p est égal à 1 et le 1,2-éthylènedioxybenzène (1,4-benzodioxole), le 3-méthyl-1,2-éthylènedioxybenzène, le 3-chloro-1,2-éthylènedioxybenzène, etc... lorsque p est égal à 2.

A titre de cétone à utiliser pour préparer de manière prédominante un composé phénolique substitué en position para selon la présente invention, les cétones citées ci-dessus peuvent être mentionnées.

A titre de cétone à utiliser de préférence selon la présente invention, on peut mentionner une monocétone aliphatique linéaire ou ramifiée ou une monocétone cyclique, et de manière plus préférée, une monocétone aliphatique linéaire ou ramifiée et parmi celles-ci, la 3,3-diméthyl-2-butanone (dans ce qui suit, elle est décrite comme "pinacoline", qui est un nom commun), la 4-méthyl-2-pentanone : la 3,3-diméthyl-2-butanone étant particulièrement préférée.

La quantité de cétone à utiliser, le type et la quantité d'acide phosphorique à utiliser, le procédé d'addition du peroxyde au système de réaction sont identiques à ceux mentionnés ci-dessus.

Dans le procédé de préparation mentionné ci-dessus, on peut utiliser la zéolite β de type protonique de la présente invention ou au moins un métal sélectionné dans le groupe consistant en un métal alcalino-terreux (Be, Mg, Ca, Sr, Ba), un métal de transition (Fe, Co, Ni, Ru, Rh, Pd, Os, Ir, Pt), un métal du groupe 2B (Zn, Cd, Hg), un métal du groupe 3A (Al, Ga, In, Ti), un métal du groupe 4A (Sn, Pb) du tableau périodique, ou un métal du groupe des lanthanides (de Ce à Lu), etc... ledit métal étant alors supporté sur la zéolite ci-dessus.

A titre d'acide carboxylique à utiliser pour préparer de manière prédominante un composé phénolique substitué en position ortho de la présente invention, on peut par exemple mentionner un acide monocarboxylique et un acide dicarboxylique. A titre d'acide monocarboxylique, on peut par exemple mentionner un acide monocarboxylique aliphatique linéaire ou ramifié ayant de 1 à 20 atomes de carbone, de préférence de 1 à 10 atomes de carbone, et un acide monocarboxylique aromatique. Le ou les atomes d'hydrogène de ces composés peuvent être substitués par des atomes d'halogène (fluor, chlore, brome ou iode). Le nombre et la position des atomes d'halogène ne sont pas critiques, tant qu'ils ne participent pas à la réaction.

A titre d'acide monocarboxylique aliphatique linéaire, on peut mentionner par exemple, l'acide formique, l'acide acétique, l'acide monochloroacétique, l'acide dichloroacétique, l'acide trichloroacétique, l'acide monofluoroacétique, l'acide difluoroacétique, l'acide trifluoroacétique, l'acide bromoacétique, l'acide propanoïque, l'acide butanoïque, l'acide pentanoïque, l'acide hexanoïque, l'acide heptanoïque, l'acide octanoïque, l'acide nonanoïque et l'acide décanoïque.

A titre d'acide monocarboxylique aliphatique ramifié, on peut mentionner par exemple, l'acide 2-méthylpropanoïque, l'acide 2-fluoropropanoïque, l'acide 2-chloropropanoïque, l'acide 2-bromopropanoïque, l'acide 2,2-diméthylpropanoïque, l'acide 2-méthylbutanoïque, l'acide 2,2-diméthylbutanoïque, l'acide 3-méthylbutanoïque, l'acide 2,3-diméthylbutanoïque, et l'acide 3,3-diméthylbutanoïque. A titre d'acide monocarboxylique aromatique, on peut par exemple mentionner l'acide benzoïque et l'acide phénylacétique.

A titre d'acide dicarboxylique, on peut par exemple mentionner un acide dicarboxylique linéaire ou ramifié ayant de 2 à 20 atomes de carbone, de préférence de 2 à 10 atomes de carbone, ou un acide dicarboxylique aromatique. Le ou les atomes d'hydrogène de ces composés peuvent être substitués par des atomes d'halogène (fluor, chlore, brome ou iode). Le nombre et la position des atomes d'halogène ne sont pas critiques, tant qu'ils ne participent pas à la réaction. A titre d'acide dicarboxylique aliphatique linéaire, on peut mentionner par exemple, l'acide oxalique, l'anhydride acétique, l'acide 1,3-propanedicarboxylique, l'acide 1,4-butanedicarboxylique, l'acide 1,5-pentanedicarboxylique, l'acide 1,6-hexanedicarboxylique, l'acide fumarique, l'acide maléique et l'anhydride maléique. Comme acide dicarboxylique aliphatique ramifié, on peut par exemple mentionner l'acide 2-méthyl-1,3-propanedicarboxylique et l'acide 2,2-diméthyl-1,3-propanedicarboxylique. A titre d'acide dicarboxylique aromatique, on peut par exemple mentionner l'acide phtalique.

A titre d'acide carboxylique utilisé de préférence dans la présente invention, on peut mentionner un acide monocarboxylique aliphatique linéaire ayant de 1 à 10 atomes de carbone et parmi ceux-ci, l'acide acétique et l'acide propanoïque sont particulièrement préférés.

La quantité d'acide carboxylique à utiliser est de préférence, un intervalle dans lequel le rapport molaire de l'acide carboxylique à la quantité de peroxyde (acide carboxylique:peroxyde) est de 0,2:1 à 5:1.

Le type et la quantité d'acide phosphorique à utiliser sont identiques à ceux mentionnés ci-dessus.

A titre de peroxyde à utiliser pour préparer de manière prédominante un composé phénolique substitué en position ortho de la présente invention, on peut citer un peroxyde inorganique comme le peroxyde d'hydrogène, etc... ou un peroxyde d'acide carboxylique.

A titre de peroxyde d'acide carboxylique, on peut par exemple citer, ceux ayant de 2 à 20 atomes de carbone, de préférence ceux ayant de2 à 10 atomes de carbone. Parmi de tels composés, on peut mentionner par exemple le peroxyde d'acide acétique, le peroxyde d'acide propanoïque, le peroxyde d'acide butanoïque, le peroxyde d'acide 2-méthylpropanoïque, le peroxyde d'acide 2-fluoropropanoïque, le peroxyde d'acide 2-chloropropanoïque, le peroxyde d'acide 2-bromopropanoïque, le peroxyde d'acide 2,2-diméthylpropanoïque, le peroxyde d'acide 2-méthylbutanoïque, le peroxyde d'acide 2,2-diméthylbutanoïque, le peroxyde d'acide 3-méthylbutanoïque, le peroxyde d'acide 2,3-diméthylbutanoïque, le peroxyde d'acide 3,3-diméthylbutanoïque et leurs analogues structuraux.

En ce qui concerne le peroxyde d'hydrogène, on peut utiliser au moins 0,1 % en poids, de préférence de 0,1 à 90 % en poids de peroxyde d'hydrogène aqueux ; la quantité de 30 à 80 % en poids étant plus particulièrement préférée.

A titre de peroxyde à utiliser pour préparer de manière prédominante, un composé phénolique substitué en

position ortho de la présente invention, le peroxyde d'hydrogène aqueux ou un peroxyde d'acide carboxylique ayant de 2 à 10 atomes de carbone est utilisé de préférence ; le peroxyde d'hydrogène aqueux à une concentration de 30 à 80 % en poids, le peroxyde d'acide acétique ou le peroxyde d'acide propanoïque étant utilisés de manière plus préférée. Le peroxyde d'acide carboxylique peut être synthétisé par mise en contact d'un acide carboxylique avec le peroxyde d'hydrogène, et l'acide carboxylique utilisé ici est le même que ceux mentionnés ci-dessus.

La quantité de peroxyde à utiliser et le procédé d'addition du peroxyde au système de réaction sont identiques à ceux décrits ci-dessus.

A titre de zéolite acide à utiliser pour préparer de manière prédominante un composé phénolique substitué en position ortho de la présente invention, on peut utiliser un matériau de type protonique de différentes zéolites comme X, Y, ZSM-5, ZSM-11, la mordénite, la chabazite, la β, etc..., et de préférence on utilise la zéolite β de type protonique conforme à la présente invention.

En tant que procédé d'addition du peroxyde au système de réaction, on peut ajouter toute la quantité de celui-ci au moment de l'initiation de la réaction, et pour prévenir l'apparition de réactions secondaires et pour effectuer la réaction avec un rendement élevé, il est préférable d'ajouter le peroxyde en plusieurs fois en le divisant en plusieurs fractions ou de l'ajouter de manière continue en faible quantité à l'aide d'une pompe d'alimentation de liquide telle qu'une pompe plongeuse. Lorsque le peroxyde est ajouté en plusieurs fois en le divisant en plusieurs fractions, la quantité de peroxyde basée sur la quantité de composé benzénique par unité de temps est de préférence de 0,05:100 à 2:100, de manière plus préférée de 0,1:100 à 2:100 en termes de rapport molaire (peroxyde:composé benzénique). De même, pour l'addition divisée ou l'addition continue, la quantité du peroxyde dans le système de réaction, sur la base de la quantité de composé benzénique, est de préférence située dans l'intervalle allant de 0,1:100 à 1:100 en termes de rapport molaire.

La zéolite β de type protonique de la présente invention à utiliser pour préparer le composé phénolique indiqué ci-dessus, peut se présenter sous la forme d'une poudre, d'un granulé, d'extrudats, d'un matériau moulé de type alvéolaire, etc.

Le composé phénolique mentionné ci-dessus, se présente de préférence sous forme d'une poudre, d'un granulé, etc. lorsque l'on utilise un réacteur à système discontinu en phase liquide pour la préparation, et de préférence sous forme d'une pastille, d'un matériau moulé de type alvéolaire, etc... lorsque l'on utilise un réacteur de système à écoulement en phase liquide pour la préparation.

Au cours de la préparation du composé phénolique mentionné ci-dessus, la quantité de la zéolite β de type protonique à utiliser, sur la base de la quantité de composé benzénique, est de préférence comprise entre 1:1 et 1:500 inclusivement, de manière plus préférée cette quantité est comprise entre 1:5 et 1:100 inclusivement, cette quantité étant exprimée en termes de rapport pondéral de la zéolite β de type protonique au composé benzénique (zéolite β: composé benzénique).

La température de réaction pour la préparation du composé phénolique indiqué ci-dessus est de préférence comprise entre 20 et 300°C, de manière plus préférée entre 40 et 200°C. La durée de réaction peut varier en fonction, par exemple, de la différence du rapport Al:Si (rapport atomique) de la zéolite β de type protonique ou de la température de réaction, mais n'est pas critique. De même, la réaction peut être réalisée sous pression atmosphérique, mais également sous pression réduite ou sous pression. La réaction peut être réalisée en phase liquide par un système discontinu, un système à écoulement, un système à lit de ruissellement.

A titre d'exemple de procédé de préparation du composé phénolique indiqué ci-dessus, on peut mentionner un procédé dans lequel un composé benzénique, le peroxyde d'hydrogène, une cétone et/ou un acide carboxylique et un acide phosphorique sont amenés dans un réacteur dans lequel la zéolite β de type protonique a été préalablement disposée, pour oxyder le composé benzénique pour conduire à un composé phénolique, puis dans lequel le mélange réactionnel est ensuite extrait du réacteur.

Le composé phénolique obtenu par le procédé de préparation ci-dessus du composé phénolique, peut être obtenu en tant que composé unique ou que mélange de plusieurs types de composés en fonction de la structure du composé benzénique utilisé comme matériau de départ. De même, ces composés phénoliques peuvent être obtenus par séparation et purification de manière classique.

Dans ce qui suit, la présente invention va être décrite spécifiquement en se référant aux exemples et exemples comparatifs. La présente invention n'est pas limitée à ces exemples.

Le rendement en phénol dihydroxylé est obtenu selon l'équation suivante. Les analyses sont réalisées par chromatographie en phase gazeuse.

Rendement en phénol dihydroxylé (%) = {(nombre de mole de phénol dihydroxylé formé) / (nombre de mole de peroxyde chargé)} x 100.

**EXEMPLE 1 : Préparation de H/β $HNO_3$ (38)**

Dans un flacon de 40 ml, dans lequel on a disposé une solution aqueuse d'acide nitrique (pH = 1,35), on dispose 5,0 g de poudre de zéolite β de type protonique ayant un rapport Al:Si égal à 1:38 (indiquée ci-après H/β (38)), disponible chez ZEOLYST INTERNATIONAL. On chauffe le mélange à 80°C à l'aide d'un bain d'huile et on agite. Après 2 heures, on élimine la zéolite par filtration, le filtrat est lavé avec de l'eau déminéralisée, séché à 110°C et calciné à 550°C à l'air pendant 2,5 heures. Lorsque la zéolite β de type protonique résultante (ci-après dénommée H/β $HNO_3$ (38)) est analysée par spectroanalyse d'émission atomique ICP, le rapport Al:Si (rapport atomique) de la zéolite β est de 1:78. De même, lorsque l'on réalise la mesure TPD-$NH_3$ (vitesse d'augmentation de la température : 20°C/minute, intervalle de mesure : 100°C à 700°C), le pic de désorption appartenant au site acide fort à une température d'au moins 500°C est nettement diminué (voir Fig. 2, courbe basse) par comparaison à celui pour lequel aucun traitement n'a été réalisé (voir Fig. 2 courbe haute). Pour le spectre TPD-$NH_3$ représenté sur cette figure, lorsque la quantité de site acide fort existant dans une région de température d'au moins 500°C est calculée à partir de l'équation 1 ci-dessus, elle est de 0,55 μmole/g, alors que sans traitement à l'acide nitrique (H/β $HNO_3$ (38)), elle est de 3,0 μmoles/g, ce par quoi on admet une différence remarquable. De même, lorsque l'on compare le spectre IR-TF, on ne détecte pas l'absorption à 3782 $cm^{-1}$, qui a été détectée pour le β (38) (voir Fig. 3).

**EXEMPLE 2 : Préparation de H/β $HNO_3$ (13)**

Dans un flacon de 40 ml, dans lequel on a disposé une solution aqueuse d'acide nitrique (pH = 1,30), on dispose 5,0 g de poudre de zéolite β de type protonique ayant un rapport Al:Si égal à 1:13 (indiquée ci-après H/β (13)), disponible chez ZEOLYST INTERNATIONAL. On chauffe le mélange à 80°C à l'aide d'un bain d'huile et on agite. Après 2 heures, on élimine la zéolite par filtration, le filtrat est lavé avec de l'eau déminéralisée, séché à 110°C et calciné à 550°C à l'air pendant 2,5 heures. Lorsque la zéolite β de type protonique résultante (ci-après dénommée H/β $HNO_3$ (13)) est analysée par spectroanalyse d'émission atomique ICP, le rapport Al:Si (rapport atomique) de la zéolite β est de 1:69. De même, lorsque l'on réalise la mesure TPD-$NH_3$ (vitesse d'augmentation de la température : 20°C/minute, intervalle de mesure : 100°C à 700°C), le pic de désorption appartenant au site acide fort à la température de 500°C ou plus est nettement diminué par comparaison à celui pour lequel aucun traitement n'a été réalisé (H/β (13)). La quantité de site acide fort calculée de la même manière qu'à l'exemple 1 est de 1,8 μmoles/g dans H/β $HNO_3$ (13) ; elle est de 4,2 μmoles/g dans H/β (13), ce par quoi on admet une différence remarquable.

De même, lorsque l'on compare le spectre IR-TF, on ne détecte pas l'absorption à 3782 $cm^{-1}$, qui a été détectée pour le H/β (13).

**EXEMPLE 3 :**

Dans un flacon de 300 ml, on dispose 0,20 g de H/β $HNO_3$ (38) tel que préparé ci-dessus à l'exemple 1, 10,0 g de phénol, 0,27 g de 3-pentanone et 0,02 g d'une solution aqueuse d'acide phosphorique à 85%. Après avoir remplacé l'atmosphère par de l'azote, on augmente la température du mélange jusqu'à 60°C sous agitation. Ensuite, à la même température, on ajoute au mélange et goutte à goutte, 0,10 g de peroxyde d'hydrogène aqueux à 60 % en poids., On ajoute ensuite goutte à goutte, 0,10 g de ce même peroxyde d'hydrogène après 1,5 minutes et encore 0,10 g après 3 minutes, puis on réalise la réaction pendant 5 minutes depuis la première addition goutte à goutte.

Il en résulte un rendement en phénol dihydroxylé de 52,8 % pour le catéchol et de 41,5% pour l'hydroquinone, avec un rendement global en catéchol et hydroquinone de 94,3%.

**EXEMPLE 4 :**

On réalise la réaction de la même manière qu'à l'exemple 3, excepté que l'on modifie la température de réaction à 100°C.

Il en résulte un rendement en phénol global en catéchol et hydroquinone de 96,0 %.

EXEMPLE COMPARATIF 1 :

On réalise la réaction de la même manière qu'à l'exemple 3, sauf que l'on remplace la H/β $HNO_3$ (38) par la H/β (38).

Il en résulte un rendement en phénol dihydroxylé de 50,0 % pour le catéchol et de 34,7 % pour l'hydroquinone, avec un rendement global en catéchol et hydroquinone de 84,7 %.

EXEMPLE COMPARATIF 2 :

On réalise la réaction de la même manière qu'à l'exemple comparatif 1, sauf que l'on modifie la température de réaction à 100°C.

Il en résulte un rendement en phénol dihydroxylé de 51,4 % pour le catéchol et de 33,6 % pour l'hydroquinone, avec un rendement global en catéchol et hydroquinone de 85,0 %.

**EXEMPLE 5 :**

On réalise la réaction de la même manière qu'à l'exemple 4, sauf que l'on remplace la H/β $HNO_3$ (38) par la H/β $HNO_3$ (13).

Il en résulte un rendement en phénol dihydroxylé de 52,6 % pour le catéchol et de 39,1 % pour l'hydroquinone, avec un rendement global en catéchol et hydroquinone de 91,7 %.

EXEMPLE COMPARATIF 3

On réalise la réaction de la même manière qu'à l'exemple 4, sauf que l'on remplace la H/β $HNO_3$ (38) par la H/β (13).

Il en résulte un rendement en phénol dihydroxylé de 49,3 % pour le catéchol et de 33,8 % pour l'hydroquinone, avec un rendement global en catéchol et hydroquinone de 83,1 %.

**Tableau 1**

| | Zéolite β de type protonique | Température de réaction (°C) | Quantité de site acide fort (μmole/g) | Rendement en phénol dihydroxylé (%) |
|---|---|---|---|---|
| Exemple 3 | H/β $HNO_3$ (38) | 60 | 0,55 | 94,3 |
| Exemple 4 | H/β $HNO_3$ (38) | 100 | 0,55 | 96,0 |
| Ex. comp. 1 | H/β (38) | 60 | 3,0 | 84,7 |
| Ex. comp. 2 | H/β (38) | 100 | 3,0 | 85,0 |
| Exemple 5 | H/β $HNO_3$ (13) | 100 | 1,8 | 91,7 |
| Ex. comp. 3 | H/β (13) | 100 | 4,2 | 83,1 |
| Remarque : H/β $HNO_3$ (38) : zéolite β de type protonique avec Al:Si = 1:38 traitée par l'acide nitrique ; H/β (38) : zéolite β de type protonique avec Al:Si = 1:38 ; H/β $HNO_3$ (13) : zéolite β de type protonique avec Al:Si = 1:13, traitée par l'acide nitrique ; H/β (13) : zéolite β de type protonique avec Al:Si = 1:13. | | | | |

**EXEMPLE 6 :**

Dans un flacon de 300 ml, on dispose 0,20 g de H/β $HNO_3$ (38) préparé à l'exemple 1, 10,0 g de phénol, et 0,27 g de 3-pentanone. Après avoir remplacé l'atmosphère par de l'azote, on augmente la température du mélange jusqu'à 100°C sous agitation. Ensuite, à la même température, on ajoute goutte à goutte, 0,10 g de peroxyde d'hydrogène aqueux à 60 % en poids au mélange, puis on ajoute goutte à goutte, 0,10 g du même peroxyde d'hydrogène après 1,5 minutes, et encore 0,10 g après 3 minutes, 0,10 g après 4,5 minutes, 0,10 g après 6 minutes et 0,10 g après 7,5 minutes. On réalise la réaction pendant 10 minutes depuis la première addition goutte à goutte de peroxyde d'hydrogène.

Il en résulte un rendement en phénol dihydroxylé de 48,4 % pour le catéchol et de 43,7 % pour l'hydroquinone, avec un rendement global en catéchol plus hydroquinone de 92,1 %.

**EXEMPLE 7 :**

On réalise la réaction de la même manière qu'à l'exemple 6, sauf que l'on ajoute 0,02 g d'une solution aqueuse d'acide phosphorique à 85 % aux matériaux de départ avant la réaction.

Il en résulte un rendement en phénol dihydroxylé de 50,4 % pour le catéchol et de 40,4 % pour l'hydroquinone, avec un rendement global en catéchol et hydroquinone de 90,8 %.

EXEMPLE COMPARATIF 4

On réalise la même réaction qu'à l'exemple 6, sauf que l'on modifie la H/β $HNO_3$ (38) par la H/β (38).

Il en résulte un rendement en phénol dihydroxylé de 45,1 % pour le catéchol et de 38,0 % pour l'hydroquinone, avec un rendement global en catéchol et hydroquinone de 83,1 %.

EXEMPLE COMPARATIF 5

On réalise la même réaction qu'à l'exemple comparatif 7, sauf que l'on remplace la H/β $HNO_3$ (38) par la H/β (38).

Il en résulte un rendement en phénol dihydroxylé de 47,7 % pour catéchol et de 33,0 % pour l'hydroquinone, avec un rendement global en catéchol et hydroquinone de 80,7 %.

**Tableau 2**

| | Zéolite β de type protonique | Quantité de site acide fort (μmole/g) | Addition d'acide phosphorique | Rendement en phénol dihydroxylé (%) |
|---|---|---|---|---|
| Exemple 6 | H/β $HNO_3$ (38) | 0,55 | Non | 92,1 |
| Exemple 7 | H/β $HNO_3$ (38) | 0,55 | Oui | 90,8 |
| Ex. comp. 4 | H/β (38) | 3,0 | Non | 83,1 |
| Ex. comp. 5 | H/β (38) | 3,0 | Oui | 80,7 |
| Remarque : H/β $HNO_3$ (38) : zéolite β de type protonique avec Al:Si = 1:38, traitée par l'acide nitrique ; H/β (38) : zéolite β de type protonique avec Al:Si = 1:38 | | | | |

EXEMPLE DE REFERENCE 1 : Préparation de la zéolite β de type protonique portant du calcium

Une solution aqueuse contenant des ions Ca, en une quantité de 15 ml, est préparée en dissolvant 1,8 g de nitrate de calcium tétrahydraté dans de l'eau déminéralisée. Dans la solution aqueuse résultante, on immerge 2 g de la zéolite β de type protonique préparée à l'exemple 1 et on maintient le mélange à 85°C pendant 14 heures pour réaliser un échange d'ions de protons en ions calcium. La suspension obtenue est soumise à une filtration par aspiration, séchée à 110°C, puis calcinée à 550°C pendant 2,5 heures pour obtenir 1,9 g d'une zéolite β de type protonique portant du calcium (dénommée ci-après "Ca/H/β $HNO_3$").

Lorsque l'on réalise la spectroanalyse d'émission atomique ICP du Ca/H/β $HNO_3$ comme indiqué ci-dessus, le rapport du calcium à l'Al (rapport atomique) introduit dans ladite zéolite β est Ca/Al = 0,41.

**EXEMPLE 8**

Dans un flacon de 300 ml, on dispose 0,50 g de H/β $HNO_3$ préparé à l'exemple 1, 46,0 g (0,425 mole) d'anisole, 1,06 g (0,0106 mole) de pinacoline (3,3-diméthyl-2-butanone) et 0,02 g d'une solution aqueuse d'acide phosphorique à 85 %. Après avoir remplacé l'atmosphère par de l'azote, on augmente la température du mélange jusqu'à 120°C par un bain d'huile sous agitation. Ensuite, à la même température, on ajoute goutte à goutte au mélange, 0,10 g de peroxyde d'hydrogène aqueux à 60 % en poids, puis on ajoute goutte à goutte, chaque fois 0,10 g du même peroxyde d'hydrogène au bout de 3 minutes, 6 minutes, 9 minutes, 12 minutes et 15 minutes, ce par quoi on a ajouté au total 0,60 g (0,0106 mole), en divisant le peroxyde d'hydrogène en six portions. On réalise la réaction pendant 60 minutes depuis la première addition goutte à goutte.

Il en résulte un rendement en méthoxyphénol de 35,8 % pour l'o-méthoxyphénol et de 52,2 % pour le p-méthoxyphénol, avec un rendement total de 88,0 %. Le mélange réactionnel est légèrement coloré en rouge pâle, et l'accumulation de composants goudronneux n'est sensiblement pas observée.

**EXEMPLE 9**

On réalise la même réaction qu'à l'exemple 8, sauf que l'on utilise la Ca/H/β $HNO_3$ préparée à l'exemple de référence 1 comme catalyseur.

Il en résulte un rendement en méthoxyphénol de 35,0 % pour l'o-méthoxyphénol et de 50,2 % pour le p-méthoxyphénol, avec un rendement total de 85,2 %. Le mélange réactionnel est légèrement coloré en rouge pâle, et l'accumulation de composants goudronneux n'est sensiblement pas observée.

EXEMPLE COMPARATIF 6

On réalise la même réaction qu'à l'exemple 8, sauf que l'on utilise 0,02 g d'acide sulfurique concentré à 98 % comme catalyseur.

Il en résulte un rendement en méthoxyphénol de 21,6 % pour l'o-méthoxyphénol et de 3,8 % pour le p-méthoxyphénol, avec un rendement total de 25,4 %. Le mélange réactionnel est légèrement coloré en rouge brun, et l'accumulation de composants goudronneux est observée.

EXEMPLE COMPARATIF 7

On réalise la même réaction qu'à l'exemple 8, sauf que l'on utilise comme catalyseur du US-Y avec Al:Si = 1: 3,0, disponible chez NIKKI-UNIVERSAL CO. LTD.

Il en résulte un rendement en méthoxyphénol de 34,9 % pour l'o-méthoxyphénol et de 12,2 % pour le p-méthoxyphénol, avec un rendement total en catéchol et hydroquinone de 47,1 %. Le mélange réactionnel est légèrement coloré en rouge pâle, et l'accumulation de composants goudronneux n'est sensiblement pas observée.

EXEMPLE COMPARATIF 8

On réalise la même réaction qu'à l'exemple 8, sauf que l'on utilise comme catalyseur du TS-1 avec Ti:Si = 1: 38,0, disponible chez N.E. CHEMCAT CORPORATION, et que l'on omet la pinacoline et l'acide phosphorique.

Il en résulte un rendement en méthoxyphénol de 4,2 % pour l'o-méthoxyphénol et de 14,9 % pour le p-méthoxyphénol, avec un rendement total en catéchol et hydroquinone de 19,1 %. Le mélange réactionnel est légèrement coloré en rouge pâle, et l'accumulation de composants goudronneux n'est sensiblement pas observée.

**Tableau 3**

| | Catalyseur | Cétone | Rendement en méthoxyphénol (%) | Rapport o/p |
|---|---|---|---|---|
| Exemple 8 | H/β $HNO_3$ | Pinacoline | 88,0 | 0,69 |
| Exemple 9 | Ca/H/β $HNO_3$ | Pinacoline | 85,2 | 0,70 |
| Ex. comp. 6 | $H_2SO_4$ | Pinacoline | 25,4 | 5,68 |
| Ex. comp. 7 | US-Y | Pinacoline | 47, 1 | 2,86 |
| Ex. comp. 8 | TS-1 | - | 19,1 | 0,28 |
| Remarque : H/β $HNO_3$ : zéolite β de type protonique traitée par l'acide nitrique ; Ca/H/β $HNO_3$ : zéolite β de type protonique portant du calcium et traitée avec une solution aqueuse d'acide nitrique ; Rapport o/p : rapport de formation du composé substitué en position ortho au composé substitué en position para | | | | |

**EXEMPLE 10**

Dans un flacon de 300 ml, on dispose 0,20 g de zéolite β de type protonique traitée à l'acide nitrique et telle que préparée à l'exemple 1, 11,5 g (0,106 mole) d'anisole, 0,27 g (0,00321 mole) de diéthylcétone et 0,02 g d'une solution aqueuse d'acide phosphorique à 85 %. Après avoir remplacé l'atmosphère par de l'azote, on augmente la température du mélange jusqu'à 120°C par un bain d'huile sous agitation. Ensuite, à la même température, on ajoute goutte à goutte au mélange, 0,10 g de peroxyde d'hydrogène aqueux à 60 % en poids, puis on ajoute goutte à goutte, 0,10 g du même peroxyde d'hydrogène après 3 minutes et 0,10 g après 6 minutes, ce par quoi on a ajouté au total 0,30 g (0,00529 mole), en divisant le peroxyde d'hydrogène aqueux en trois portions. On réalise la réaction pendant 60 minutes depuis la première addition goutte à goutte.

Il en résulte un rendement en méthoxyphénol de 32,6 % pour l'o-méthoxyphénol et de 44,4 % pour le p-méthoxyphénol, avec un rendement total de 77,0 %. Le mélange réactionnel est légèrement coloré en rouge pâle, et l'accumulation de composants goudronneux n'est sensiblement pas observée.

**EXEMPLE 11**

On réalise la même réaction qu'à l'exemple 10, sauf que l'on utilise comme cétone 0,63 g (0,00629 mole) de méthylisobutylcétone (4-méthyl-2-pentanone).

Il en résulte un rendement en méthoxyphénol de 34,6 % pour l'o-méthoxyphénol et de 37,9 % pour le p-méthoxyphénol, avec un rendement total de 72,5 %. Le mélange réactionnel est légèrement coloré en rouge brun pâle, et l'accumulation de composants goudronneux n'est sensiblement pas observée.

**EXEMPLE 12**

On réalise la même réaction qu'à l'exemple 10, sauf que l'on utilise comme cétone 0,32 g (0,00319 mole) de pinacoline (3,3-diméthyl-2-butanone).

Il en résulte un rendement en méthoxyphénol de 33,5 % pour l'o-méthoxyphénol et de 46,4 % pour le p-méthoxyphénol, avec un rendement total de 79,9 %. Le mélange réactionnel est légèrement coloré en rouge brun pâle, et l'accumulation de composants goudronneux n'est sensiblement pas observée.

**Tableau 4**

| | Catalyseur | Cétone | Rendement en méthoxyphénol (%) | Rapport o/p |
|---|---|---|---|---|
| Exemple 10 | H/β $HNO_3$ | Diéthylcétone | 77,0 | 0,73 |
| Exemple 11 | H/β $HNO_3$ | Méthylisobutylcétone | 72,5 | 0,91 |
| Exemple 12 | H/β $HNO_3$ | Pinacoline | 79,9 | 0,72 |
| Remarque : H/β $HNO_3$ : zéolite β de type protonique traitée par l'acide nitrique | | | | |

**EXEMPLE 13**

On réalise la même réaction qu'à l'exemple 8, sauf que l'on utilise 52,0 g (0,425 mole) de phénétol (éthoxybenzène) comme substrat de réaction à la place de l'anisole.

Il en résulte un rendement en éthoxyphénol de 32,5 % pour l'o-éthoxyphénol et de 46,1 % pour le p-éthoxyphénol, avec un rendement total de 78,6 %. Le mélange réactionnel est légèrement coloré en rouge pâle, et l'accumulation de composants goudronneux n'est sensiblement pas observée.

**Tableau 5**

| | Catalyseur | Substrat de réaction | Cétone | Rendement en éthoxy-phénol (%) | Rapport o/p |
|---|---|---|---|---|---|
| Exemple 13 | H/β $HNO_3$ | Phénétol | Pinacoline | 78,6 | 0,71 |
| Remarque : H/β $HNO_3$ : zéolite β de type protonique traitée par l'acide nitrique | | | | | |

**EXEMPLE 14**

Dans un flacon de 300 ml, on dispose 0,50 g de H/β $HNO_3$ telle que préparée à l'exemple 1, 46,0 g (0,425 mole) d'anisole, 0,64 g (0,0107 mole) d'acide acétique et 0,02 g d'une solution aqueuse d'acide phosphorique à 85 %. Après avoir remplacé l'atmosphère par de l'azote, on augmente la température du mélange jusqu'à 100°C par un bain d'huile sous agitation. Ensuite, à la même température, on ajoute goutte à goutte au mélange, 0,10 g de peroxyde d'hydrogène aqueux à 60 % en poids, puis on ajoute goutte à goutte, 0,10 g du même peroxyde d'hydrogène au bout de 3 minutes, 6 minutes, 9 minutes, 12 minutes et 15 minutes, ce par quoi on a ajouté au total 0,60 g (0,0106 mole), en divisant le peroxyde d'hydrogène aqueux en six portions. On réalise la réaction pendant 180 minutes depuis la première addition goutte à goutte.

Il en résulte un rendement en méthoxyphénol de 48,5 % pour l'o-méthoxyphénol et de 29,3 % pour le p-méthoxyphénol, avec un rendement total de 77,8 %. Le mélange réactionnel est légèrement coloré en rouge pâle, et l'accumulation de composants goudronneux n'est sensiblement pas observée.

**EXEMPLE 15**

Dans un flacon de 300 ml, on dispose 0,20 g de Ca/H/β $HNO_3$ telle que préparée à l'exemple de référence 1, 23,0 g (0,213 mole) d'anisole, 0,32 g (0,00535 mole) d'acide acétique et 0,02 g d'une solution aqueuse d'acide phosphorique à 85 %. Après avoir remplacé l'atmosphère par de l'azote, on augmente la température du mélange jusqu'à 100°C par un bain d'huile sous agitation. Ensuite, à la même température, on ajoute goutte à goutte au mélange, 0,10

g de peroxyde d'hydrogène aqueux à 60 % en poids, puis on ajoute goutte à goutte, 0,10 g du même peroxyde d'hydrogène après 3 minutes et 6 minutes, ce par quoi on a ajouté au total 0,30 g (0,00530 mole), en divisant le peroxyde d'hydrogène aqueux en trois portions. On réalise la réaction pendant 180 minutes depuis la première addition goutte à goutte.

Il en résulte un rendement en méthoxyphénol de 49,5 % pour l'o-méthoxyphénol et de 29,9 % pour le p-méthoxyphénol, avec un rendement total de 79,4 %. Le mélange réactionnel est légèrement coloré en rouge pâle, et l'accumulation de composants goudronneux n'est sensiblement pas observée.

EXEMPLE COMPARATIF 9

On réalise la même réaction qu'à l'exemple 14 sauf que l'on remplace le catalyseur par H/β (38) et que l'on n'utilise pas d'acide acétique.

Il en résulte un rendement en méthoxyphénol de 28,0 % pour l'o-méthoxyphénol et de 12,4 % pour le p-méthoxyphénol, avec un rendement total de 40,4%.

**Tableau 6**

| | Catalyseur | Acide carboxylique | Rendement en méthoxyphénol (%) | Rapport o/p |
|---|---|---|---|---|
| Exemple 14 | H/β $HNO_3$ | Acide acétique | 77,8 | 1,66 |
| Exemple 15 | Ca/H/β $HNO_3$ | Acide acétique | 79,4 | 1,66 |
| Ex. comp. 9 | H/β | - | 40,4 | 2,25 |
| Remarque : H/β $HNO_3$ : zéolite β de type protonique traitée par l'acide nitrique ; Ca/H/β $HNO_3$: zéolite β de type protonique portant du calcium et traitée avec une solution aqueuse d'acide nitrique ; H/β : zéolite β de type protonique ; Rapport o/p : rapport de formation du composé substitué en position ortho au composé substitué en position para. | | | | |

**EXEMPLE 16**

Dans un flacon de 300 ml, on dispose 0,20 g de H/β $HNO_3$ telle que préparée à l'exemple 1, 11,5 g (0,106 mole) d'anisole, 0,20 g (0,00333 mole) d'acide acétique et 0,02 g d'une solution aqueuse d'acide phosphorique à 85 %. Après avoir remplacé l'atmosphère par de l'azote, on augmente la température du mélange jusqu'à 120°C par un bain d'huile sous agitation. Ensuite, à la même température, on ajoute goutte à goutte au mélange, 0,10 g de peroxyde d'hydrogène aqueux à 60 % en poids, puis on ajoute goutte à goutte, 0,10 g du même peroxyde d'hydrogène après 3 minutes et 6 minutes, ce par quoi on a ajouté au total 0,30 g (0,00529 mole), en divisant le peroxyde d'hydrogène aqueux en trois portions. Après 10 minutes, on augmente la température du bain d'huile à 140°C et on réalise la réaction pendant 60 minutes depuis la première addition goutte à goutte.

Il en résulte un rendement en méthoxyphénol de 39,9 % pour l'o-méthoxyphénol et de 28,5 % pour le p-méthoxyphénol, avec un rendement total de 68,4 %.

**EXEMPLE 17**

On réalise la même réaction qu'à l'exemple 16 sauf que l'on remplace l'acide carboxylique par 0,24 g (0,00324 mole) d'acide propanoïque (acide propionique).

Il en résulte un rendement en méthoxyphénol de 34,2 % pour l'o-méthoxyphénol et de 23,7 % pour le p-méthoxyphénol, avec un rendement total de 57,9 %.

**EXEMPLE 18**

On réalise la même réaction qu'à l'exemple 16 sauf que l'on remplace l'acide carboxylique par 0,28 g (0,00318 mole) d'acide 2-méthylpropanoïque (acide isobutyrique).

Il en résulte un rendement en méthoxyphénol de 28,8 % pour l' o-méthoxyphénol et de 21,3 % pour le p-méthoxyphénol, avec un rendement total de 50,1 %.

**EXEMPLE 19**

On réalise la même réaction qu'à l'exemple 16 sauf que l'on remplace l'acide carboxylique par 0,33 g (0,00323 mole) d'acide 2,2-diméthylpropanoïque (acide pivalique).

Il en résulte un rendement en méthoxyphénol de 27,7 % pour l'o-méthoxyphénol et de 22,1 % pour le p-méthoxyphénol, avec un rendement total de 49,8 %.

**Tableau 7**

| | Catalyseur | Acide carboxylique | Rendement en méthoxyphénol (%) | Rapport o/p |
|---|---|---|---|---|
| Exemple 16 | H/β $HNO_3$ | Acide acétique | 68,4 | 1,40 |
| Exemple 17 | H/β $HNO_3$ | Acide propionique | 57,9 | 1,44 |
| Exemple 18 | H/β $HNO_3$ | Acide isobutyrique | 50,1 | 1,35 |
| Exemple 19 | H/β $HNO_3$ | Acide pivalique | 49,8 | 1,25 |
| Remarque : H/β $HNO_3$ : zéolite β de type protonique traitée par l'acide nitrique | | | | |

## EXEMPLE 20

On réalise la même réaction qu'à l'exemple 14, sauf que l'on utilise 52,0 g (0,425 mole) de phénétol (éthoxybenzène) comme substrat de réaction à la place de l'anisole, la température de réaction est fixée à 120°C et la durée de réaction est de 60 minutes.

Il en résulte un rendement en éthoxyphénol de 44,3 % pour l'o-éthoxyphénol et de 29,9 % pour le p-éthoxyphénol, avec un rendement total de 74,2 %. Le mélange réactionnel est légèrement coloré en rouge pâle, et l'accumulation de composants goudronneux n'est sensiblement pas observée.

**Tableau 8**

| | Catalyseur | Substrat de réaction | Acide carboxylique | Rendement en éthoxyphénol (%) | Rapport o/p |
|---|---|---|---|---|---|
| Exemple 20 | H/β $HNO_3$ | Phénétol | Acide acétique | 74,2 | 1,48 |
| Remarque : H/β $HNO_3$ : zéolite β de type protonique traitée par l'acide nitrique | | | | | |

## EXEMPLE 21

Dans un flacon de 300 ml, on dispose 0,20 g de H/β $HNO_3$ (38) préparée à l'exemple 1, 9,80 g de toluène, 0,27 g de 3-pentanone et 0,02 g d'une solution aqueuse d'acide phosphorique à 85 %. Après avoir remplacé l'atmosphère par de l'azote, on augmente la température du mélange jusqu'à 120°C sous agitation. Ensuite, à la même température, on ajoute goutte à goutte au mélange, 0,10 g de peroxyde d'hydrogène aqueux à 60 % en poids, puis on ajoute goutte à goutte 0,10 g du même peroxyde d'hydrogène après 1,5 minute et 0,10 g après 3 minutes, et on réalise la réaction pendant 60 minutes depuis la première addition goutte à goutte.

Il en résulte un rendement en o-crésol, qui est un produit oxydé du toluène, de 16,2 % et en p-crésol, qui est également un produit oxydé du toluène, de 19,5 %, avec un rendement total en crésol de 35,7 %.

## EXEMPLE 22

On réalise la même réaction qu'à l'exemple 21, sauf que l'on remplace la 3-pentanone par 0,34 g de pinacoline (3,3-diméthyl-2-butanone).

Il en résulte un rendement en o-crésol, qui est un produit oxydé du toluène, de 20,3 % et en p-crésol, qui est également un produit d'oxydation du toluène, de 21,9 %, avec un rendement total en crésol de 42,2 %.

## EXEMPLE 23

Dans un flacon de 300 ml, on dispose 0,50 g de H/β $HNO_3$ (38) telle que préparée à l'exemple 1, 52,0 g (0,412 mole) de méthylènedioxybenzène, 2,16g (0,022 mole) de pinacoline et 0,02 g d'une solution aqueuse d'acide phosphorique à 85 %. Après avoir remplacé l'atmosphère par de l'azote, on augmente la température du mélange jusqu'à 100°C sous agitation. Ensuite, à la même température, on ajoute goutte à goutte au mélange, 0,10 g de peroxyde d'hydrogène aqueux à 60 % en poids, puis on ajoute goutte à goutte 0,10 g du même peroxyde d'hydrogène après 1,5 minute, 0,10 g après 3 minutes, 0,10 g après 4,5 minutes, 0,10 g après 6 minutes et 0,10 g après 7,5 minutes, et on réalise la réaction pendant 30 minutes depuis la première addition goutte à goutte.

Il en résulte un rendement en sésamol (3,4-méthylènedioxyphénol) de 28,0 %.

**EXEMPLE 24**

Dans un flacon de 300 ml, équipé d'un piège Dean-Stark préparé par VIDEX Co., on dispose 0,40 g de H/β $HNO_3$ (38) telle que préparée à l'exemple 1 et 15,00 g de 2,3,6-triméthylphénol. Après avoir remplacé l'atmosphère par de l'azote, on augmente la température du mélange jusqu'à 120°C sous agitation. Ensuite, à la même température, on ajoute goutte à goutte au mélange, 0,10 g de peroxyde d'hydrogène aqueux à 60 % en poids et 0,40 g de 4-méthyl-2-pentanone. Après 20 minutes, on ajoute ensuite goutte à goutte au mélange, les mêmes quantités de peroxyde d'hydrogène aqueux à 60 % en poids et de 4-méthyl-2-pentanone, et on réalise la réaction pendant 30 minutes depuis la première addition goutte à goutte. Pendant la réaction, l'eau est éliminée en continu par le piège Dean-Stark.
Il en résulte un rendement en 2,3,5-triméthylhydroquinone de 71,9 %.

EXEMPLE COMPARATIF 10 (exemple comparatif 1 supplémentaire)

On réalise la même réaction qu'à l'exemple 25, sauf que l'on remplace le catalyseur par H/β (38).
Il en résulte un rendement en 2,3,5-triméthylhydroquinone de 52,1 %.
La zéolite β de type protonique de la présente invention peut être préparée par un procédé simple et aisé en immergeant la zéolite β dans une solution acide aqueuse. La présente zéolite β de type protonique présente une réactivité élevée à une plus faible température au cours de la préparation d'un composé phénolique par oxydation d'un composé benzénique avec un peroxyde, et conduit à un composé phénolique avec un rendement élevé.

**Revendications**

1. Procédé de préparation d'au moins un des composés phénoliques choisis dans le groupe consistant en un composé représenté par les formules (3) et (4) suivantes :

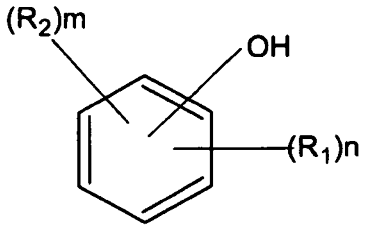

(3)

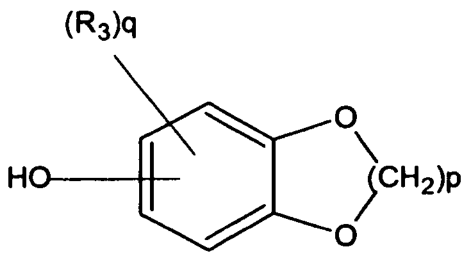

(4)

dans lesquelles :

- $R_1$ représente un radical hydroxyle, un radical alkyle linéaire ou ramifié ayant de 1 à 6 atomes de carbone ou un radical alcoxy linéaire ou ramifié ayant de 1 à 6 atomes de carbone ;
- $R_2$ représente un atome d'hydrogène, un radical alkyle linéaire ou ramifié ayant de 1 à 6 atomes de carbone, un radical alcoxy linéaire ou ramifié ayant de 1 à 6 atomes de carbone ou un atome d'halogène ;
- n est un nombre entier allant de 0 à 5 ;

- m est un nombre entier allant de 0 à 5 ;
- la somme n+m va de 0 à 5 ;
- en cas de pluralité de radicaux $R_1$ et/ou $R_2$, ceux-ci peuvent être identiques ou différents ;
- $R_3$ représente un atome d'hydrogène, un radical alkyle linéaire ou ramifié ayant de 1 à 6 atomes de carbone, un radical alcoxy linéaire ou ramifié ayant de 1 à 6 atomes de carbone ou un atome d'halogène ;
- p est un nombre entier allant de 1 à 2 et
- q est un nombre entier allant de 0 à 3 ;

qui comprend l'oxydation d'au moins un des composés benzéniques représentés par les formules (1) et (2) suivantes :

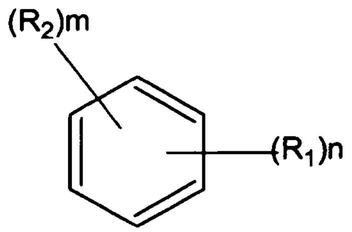

(1)

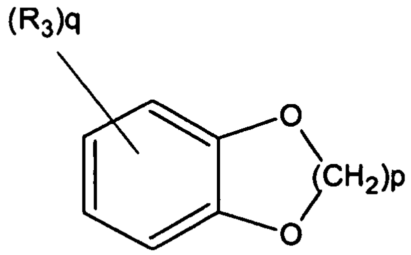

(2)

dans lesquelles $R_1$, $R_2$, $R_3$, n, m, p et q ont les mêmes significations que celles définies ci-dessus pour les composés de formules (3) et (4),
avec un peroxyde en la présence d'une zéolite β de type protonique comprenant un site acide présentant un pic de désorption dans une gamme de ± 100°C avec un centre à 330°C, selon un spectre mesuré par la méthode de désorption à l'ammoniaque à température programmée ($TPD-NH_3$), et une quantité de site acide fort présentant un pic de désorption à une température d'au moins 500°C inférieure ou égale à 2,5 μmole/g, et en présence d'une cétone ou d'un acide carboxylique.

**2.** Procédé de préparation d'un composé phénolique selon la revendication 1, dans lequel $R_1$ est un radical alcoxy ayant de 1 à 6 atomes de carbone, n est 1 et les $R_2$ restants sont des atomes d'hydrogène.

**3.** Procédé de préparation d'un composé phénolique selon la revendication 2, dans lequel la zéolite β de type protonique est une zéolite sur laquelle est supporté au moins un métal sélectionné dans le groupe consistant en un métal alcalino-terreux, un métal de transition, un métal du groupe 2B, Zn, Cd, Hg, un métal du groupe 3A, de préférence A1, Ga, In et Ti, un métal du groupe 4A, Sn et Pb du tableau périodique et un métal du groupe des lanthanides, de préférence de Ce à Lu

**4.** Procédé de préparation d'un composé phénolique selon la revendication 2, dans lequel le peroxyde est du peroxyde d'hydrogène ou un peroxyde de cétone.

**5.** Procédé de préparation d'un composé phénolique selon la revendication 4, dans lequel la réaction est réalisée en présence d'acide phosphorique.

**6.** Procédé de préparation d'un composé phénolique selon la revendication 2, dans lequel le peroxyde est du peroxyde d'hydrogène ou un peroxyde d'acide carboxylique.

**7.** Procédé de préparation d'un composé phénolique selon la revendication 6, dans lequel la réaction est réalisée en

présence d'acide phosphorique.

**8.** Procédé de préparation d'un composé phénolique selon la revendication 1, dans lequel $R_1$ est un radical hydroxyle, n est 1 et au moins un des $R_2$ est un radical alkyle linéaire ou ramifié ayant de 1 à 6 atomes de carbone, ou un atome d'halogène.

**9.** Procédé de préparation d'un composé phénolique selon la revendication 8, dans lequel le peroxyde est du peroxyde d'hydrogène ou un peroxyde de cétone.

## Claims

**1.** A process for preparing at least one of phenol compounds selected from the group consisting of a compound represented by the following formulae (3) and (4):

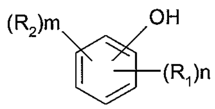

(3)

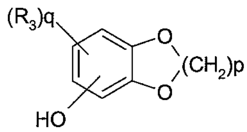

(4)

wherein

- $R_1$ represents a hydroxyl group, a straight or branched alkyl group having 1 to 6 carbon atoms or a straight or branched alkoxy group having 1 to 6 carbon atoms;
- $R_2$ represents a hydrogen atom, a straight or branched alkyl group having 1 to 6 carbon atoms, a straight or branched alkoxy group having 1 to 6 carbon atoms or a halogen atom;
- n is an integer of 0 to 5;
- m is an integer of 0 to 5;
- n + m = 0 to 5;
- in the case of a plural number of $R_1$ and/or $R_2$ radicals, these radicals may be the same or different from each other;
- $R^3$ represents a hydrogen atom, a straight or branched alkyl group having 1 to 6 carbon atoms, a straight or branched alkoxy group having 1 to 6 carbon atoms or a halogen atom;
- p is an integer of I to 2 and
- q is an integer of 0 to 3;

which comprises oxidizing at least one of benzene compounds represented by the following formulae (1) and (2):

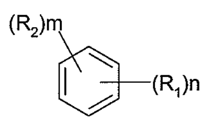

(1)

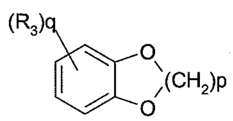

(2)

wherein $R_1$, $R_2$, $R_3$, n, m, p and q have the same meanings as defined above for compounds of formulae (3) and (4),

with a peroxide in the presence of a proton type β zeolite comprising an acid site showing a desorption peak with a range of ±100°C with a center of 330°C according to a spectrum measured by the ammonia temperature programmed desorption method ($NH_3$ -TPD), and an amount of a strong acid site showing a desorption peak at a temperature of at least 500°C which is less than or equal to 2.5 μmol/g, and in the presence of a ketone or a carboxylic acid.

**2.** The process for preparing a phenol compound according to Claim 1, wherein $R_1$ is an alkoxy group having 1 to 6 carbon atoms, n is 1 and the remaining $R_2$ are hydrogen atoms.

**3.** The process for preparing a phenol compound according to Claim 2, wherein the proton type β zeolite is a zeolite on which at least one metal selected from the group consisting of an alkaline earth metal, a transition metal, a group 2B metal, preferably Zn, Cd, Hg, a group 3A metal, preferably Al, Ga, In and Ti, a group 4A metal, preferably Sn and Pb, of the periodic table and a lanthanide group metal, preferably from Ce to Lu, is carried.

**4.** The process for preparing a phenol compound according to Claim 2, wherein the peroxide is hydrogen peroxide or a ketone peroxide.

**5.** The process for preparing a phenol compound according to Claim 4, wherein the reaction is carried out in the presence of phosphoric acid.

**6.** The process for preparing a phenol compound according to Claim 2, wherein the peroxide is hydrogen peroxide or a carboxylic acid peroxide.

**7.** The process for preparing a phenol compound according to Claim 6, wherein the reaction is carried out in the presence of phosphoric acid.

**8.** The process for preparing a phenol compound according to Claim 1, wherein the $R_1$ is a hydroxyl group, n is 1 and at least one of $R_2$ is a straight or branched alkyl group having 1 to 6 carbon atoms, or a halogen atom.

**9.** The process for preparing a phenol compound according to Claim 8, wherein the peroxide is hydrogen peroxide or a ketone peroxide.

## Patentansprüche

**1.** Verfahren zur Herstellung von mindestens einer der Phenolverbindungen, die aus der Gruppe ausgewählt werden, die aus einer Verbindung besteht, die durch die folgenden Formeln (3) und (4) dargestellt wird:

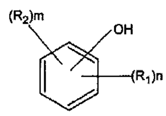

(3)

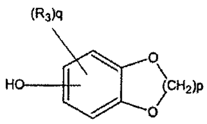

(4)

in denen:

- $R_1$ ein Hydroxylradikal, ein lineares oder verzweigtes Alkylradikal mit 1 bis 6 Kohlenstoffatomen oder ein lineares oder verzweigtes Alkoxyradikal mit 1 bis 6 Kohlenstoffatomen darstellt:
- $R_2$ ein Wasserstoffradikal, ein lineares oder verzweigtes Alkylradikal mit 1 bis 6 Kohlenstoffatomen, ein lineares oder verzweigtes Alkoxyradikal mit 1 bis 6 Kohlenstoffatomen oder ein Halogenatom darstellt:
- n eine ganze Zahl zwischen 0 und 5 ist:

- m eine ganze zahl zwischen 0 und 5 ist:
- die Summe n+m von 0 bis 5 geht:
- für den Fall, dass mehrere Radikale $R_1$ und/oder $R_2$ vorliegen, diese gleich oder verschieden sein können:
- $R_3$ ein Wasserstoffradikal, ein lineares oder verzweigtes Alkylradikal mit 1 bis 6 Kohlenstoffatomen, ein lineares oder verzweigtes Alkoxyradikal mit 1 bis 6 Kohlenstoffatomen oder ein Halogenatom darstellt:
- p eine ganze Zahl zwischen 1 und 2 ist und
- q eine ganze Zahl zwischen 0 und 3 ist;

das die Oxidation von mindestens einem der Benzenverbindungen umfasst, die durch die folgenden Formeln (1) und (2) dargestellt werden:

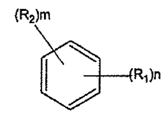
(1)

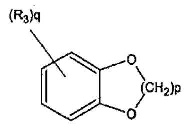
(2)

in denen $R_1$, $R_2$, $R_3$, n, m, p und q dieselben Bedeutungen haben, wie die, die oben für die Verbindungen mit den Formeln (3) und (4) definiert worden sind,
mit einem Peroxyd in Gegenwart eines Zeoliths β vom Proton-Typ umfassend einen Säureort und einen Desorptionespic in einem Bereich von +/- 100°C mit einem Zentrum bei 330°C gemäß einem Spektrum, das durch das Ammoniakdesorptionsverfahren mit programmierter Temperatur (TPD-$NH_3$) gemessen worden ist, und eine Quantität an Orten mit starken Säuren, die einen Desorptionspic bei einer Temperatur von mindestens 500°C von unter oder gleich 2,5 μmol/g bei Gegenwart eines Ketons oder einer Carboxylsäure aufweist.

2. Verfahren zur Herstellung einer Phenolverbindung nach Anspruch 1, in dem $R_1$ ein Alkoxyradikal mit 1 bis 6 Kohlenstoffatomen ist, n gleich 1 und die verbleibenden $R_2$ Wasserstoffatome sind.

3. Verfahren zur Herstellung einer Phenolverbindung nach Anspruch 2, in dem das Zeoliths β vom Proton-Typ ein Zeolith ist, auf dem mindestens ein Metall aufgetragen ist, das aus der Gruppe ausgewählt wird, das aus einem Erdalkalimetall, einem Übergangsmetall, einem Metall aus der Gruppe 2B, bevorzugt Zn, Cd, Hg, einem Metall aus der Gruppe 3A, bevorzugt Al, Ga, In und Ti, einem Metall aus der Gruppe 4A, bevorzugt Sn und Pb, des periodischen Systems und einem Metall aus der Gruppe Lanthaniden, bevorzugt von Ce bis Lu besteht.

4. Verfahren zur Herstellung einer Phenolverbindung nach Anspruch 2, in dem das Peroxyd in Wasserstoffperoxyd oder ein Ketonperoxyd ist.

5. Verfahren zur Herstellung einer Phenolverbindung nach Anspruch 4, in dem die Reaktion in Gegenwart von Phosphorsäure ausgeführt wird.

6. Verfahren zur Herstellung einer Phenolverbindung nach Anspruch 2, in dem das Peroxyd in Wasserstoffperoxyd oder ein Carboxylsäureperoxyd ist.

7. Verfahren zur Herstellung einer Phenolverbindung nach Anspruch 6, in dem die Reaktion in Gegenwart von Phosphorsäure ausgeführt wird.

8. Verfahren zur Herstellung einer Phenolverbindung nach Anspruch 1, in dem $R_1$ ein Hydroxylradikal mit 1 bis 6 Kohlenstoffatomen ist, n gleich 1 ist und mindestens einer der $R_2$ ein lineares oder verzweigtes Alkylradikal mit 1 bis 6 Kohlenstoffatomen oder ein Halogenatom ist.

9. Verfahren zur Herstellung einer Phenolverbindung nach Anspruch 8, in dem das Peroxyd ein Wasserstoffperoxyd

oder ein Ketonperoxyd ist.

Fig. 1

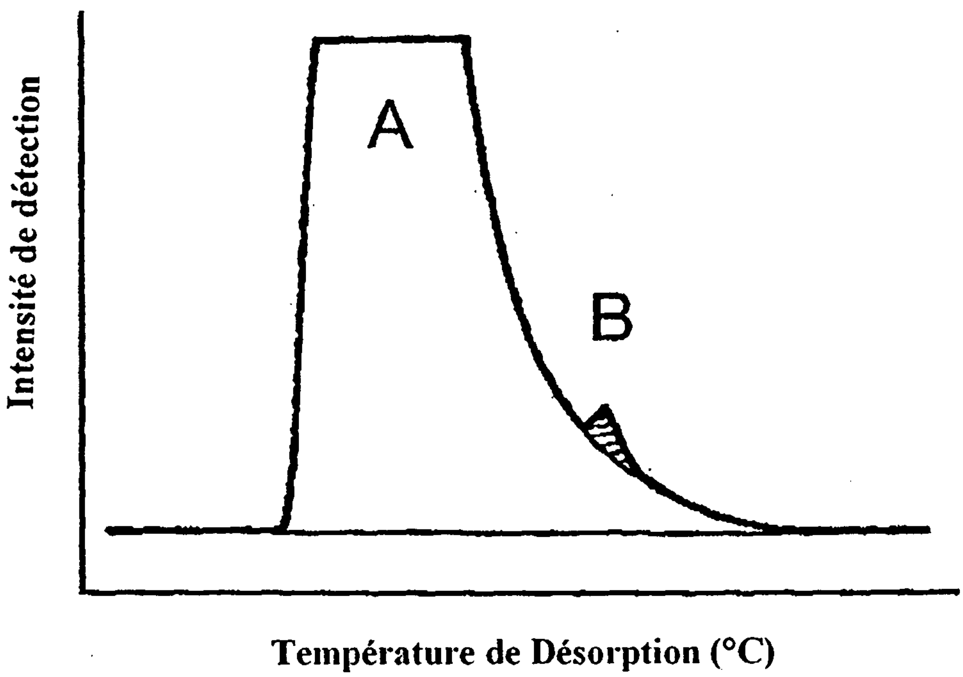


A: Pic principal avec centre à 330°C

B: Pic de désorption de 500°C ou plus

Fig. 2

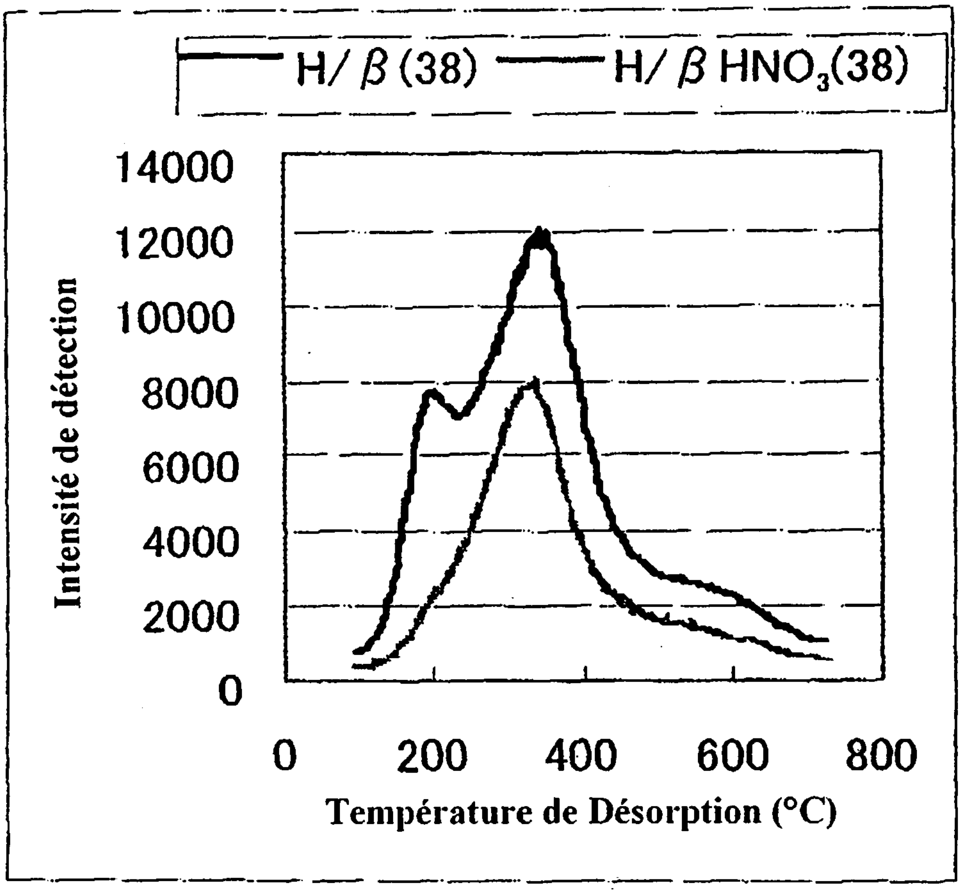
H/β(38)
H/β HNO3(38)
14000
12000
10000
8000
6000
4000
2000
0
0
200
400
600
800
Intensité de détection
Température de Désorption (°C)

Fig. 3

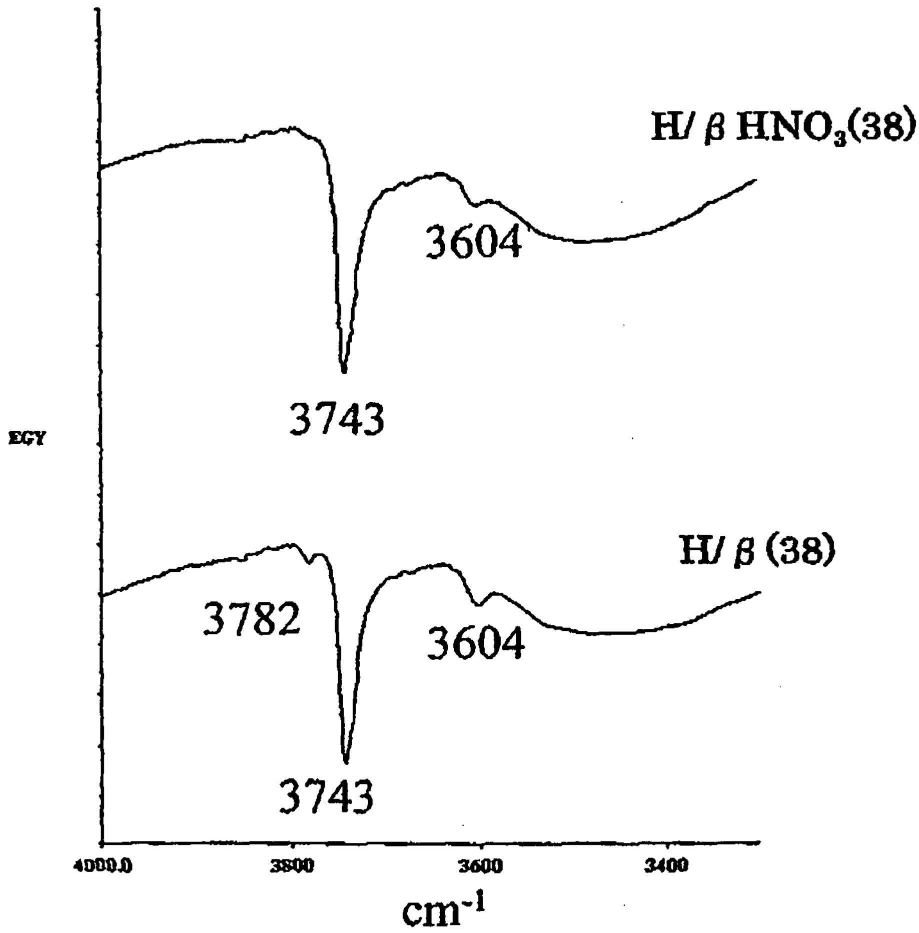
H/ β HNO3(38)
3604
3743
EGY
H/ β (38)
3782
3604
3743
4000.0
3800
3600
3400
cm-1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 9151151 A
- US 3580956 A
- US 4578521 A
- FR 2693457
- US 6441250 B
- JP 2003026623 A
- JP 3128336 A
- US 6025293 A


### Littérature non-brevet citée dans la description


- 13901 Chemicals. The Chemical Daily Co., Ltd, 2001, 653
- *Advances in Catalysis,* 1996, vol. 41, 253-334
- *Accounts of Chemical Research,* 1998, vol. 31 (8), 485-493
- *Journal of Physical Chemistry,* 2001, vol. 203, 201-212
- *Journal of American Chemical Society,* 1981, vol. 103, 3045-3049
- *Journal of Chemical Society Perkin Transition,* 1990, vol. 1 (6), 863-867
- *Journal of Chemical Society, Chemical Communications,* 1995, vol. 3, 349-350
- 13901 Chemicals. The Chemical Daily Co, 2001, 653
- *Preparations and Procedures Int.,* 2000, vol. 32, 373-405
- *le Journal of Chemical Society, Chemical Communications,* 1995, vol. 3, 349-350
- **ROBERGE et al.** *Physical Chemistry Chemical Physics,* 2002, vol. 13, 3128-3135
- *Journal of Physical Chemistry,* 2000, vol. 104, 2853-2859
- **KODANSHA.** *Catalyst Préparation Chemistry,* 1980, 61-73
- *Microporous and Mesoporous Materials,* 2000, vol. 40, 271-281